# EUROPEAN PATENT SPECIFICATION

(11) **EP 2 149 555 B1**
(45) Date of publication and mention of the grant of the patent: **14.12.2011**
(21) Application number: 09251918.0
(22) Date of filing: 30.07.2009
(51) Int. Cl.: C07D 209/86, C07D 401/12, C09K 11/06

(54) **Amine-based compound, organic light emitting device comprising the amine-based compound, and flat panel display device including the organic light emitting device**
Aminbasierte Verbindung, organische lichtemittierende Vorrichtung mit der aminbasierten Verbindung und Flachbildschirmanzeigenvorrichtung mit der organischen lichtemittierenden Vorrichtung
Composé à base d'amine, dispositif électroluminescent organique comprenant le composé à base d'amine, et dispositif à écran plat incluant le dispositif électroluminescent organique

(30) Priority: 30.07.2008 KR 20080074713
(43) Date of publication of application: 03.02.2010
(73) Proprietor: Samsung Mobile Display Co., Ltd., Yongin-city, Gyunggi-do 446-711 (KR)
(72) Inventor: Hwang, Seok-Hwan, Yongin-city, Gyunggi-do 446-711 (KR); Kawk, Yoon-Hyun, Yongin-city, Gyunggi-do 446-711 (KR); Kim, Young-Kook, Yongin-city, Gyunggi-do 446-711 (KR); Yi, Jeoung-In, Yongin-city, Gyunggi-do 446-711 (KR); Lee, Jong-Hyuk, Yongin-city, Gyunggi-do 446-711 (KR)
(74) Representative: Taor, Simon Edward William

(56) References cited:
- WO-A-2007/007885
- WO-A-2008/062636
- WO-A-2009/084268
- US-A1- 2005 221 124

## Description

### BACKGROUND OF THE INVENTION

### 1. Field of the Invention

The present invention relates to amine-based compounds and organic light emitting devices with an organic film including the same.

### 2. Description of the Related Art

Organic light emitting diodes are self light-emitting devices that have wide viewing angles, excellent contrast, and quick response time, and thus have received a lot of attention. In addition, organic light emitting diodes have high luminance, low operating voltage, and can display a full range of color. Therefore, research in organic light emitting diodes continues to grow.

Organic light emitting diodes conventionally have an anode/organic layer/cathode structure. The organic layer may include at least one of a hole injection layer, a hole transport layer, an emissive layer, an electron transport layer, and an electron injection layer.

Materials for forming the hole injection layer and the hole transport layer include polyphenyl compounds, and anthracene derivatives. However, organic light emitting diodes including a hole injection layer and/or a hole transport layer formed from conventional materials do hot have satisfactory life span, efficiency, and power consumption, thus leaving room for improvement.

WO 2008/062636 discloses an aromatic amine derivative having a specific structure having a carbazole skeleton to which a diarylamino group has been attached through a linking group. There is also provided an organic electroluminescent element comprising an organic thin-film layer, comprising at least a luminescent layer, held between a cathode and an anode.

US 2005/0221124 discloses an organic electroluminescent compound that comprises at least one fluroene derivative and at least one carbazole derivative.

WO 2007/007885 discloses a carbazole derivative that is aimed for use of a raw material in manufacturing a light emitting element material.

### SUMMARY OF THE INVENTION

The present invention provides an amine-based compound according to Claim 1, an organic light emitting device according to Claim 11, and a flat panel display device according to Claim 17. Preferred features are set out in the associated dependent claims in each case.

According to a first aspect of the present invention, an amine-based compound represented by Formula 1 is provided. 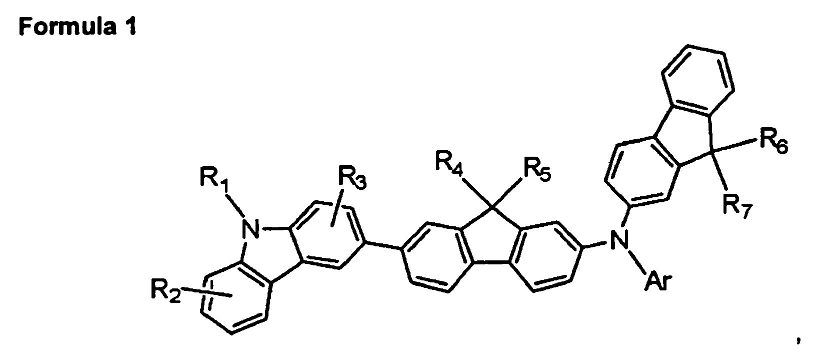 where Ar is a substituted or unsubstituted C₆-C₃₀ aryl group, a substituted or unsubstituted C₆-C₂₀ aryloxy group, a substituted or unsubstituted C₄-C₂₀ heteroaryl group, or a substituted or unsubstituted C₄-C₂₀ condensed polycyclic group,
R₁ is hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ heteroaryl group, or a substituted or unsubstituted C₄-C₂₀ condensed polycyclic group,
each of R₂ and R₃ is independently hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ condensed polycyclic group, fluorine, a cyano group, or an amino group, and
each of R₄, R₅, R₆, and R₇ is independently hydrogen, a substituted or unsubstituted C₁-C₂₀ alkyl group, a substituted or unsubstituted C₁-C₂₀ alkoxy group, or a substituted or unsubstituted C₆-C₃₀ aryl group.

According to another aspect of the present invention, an organic light emitting device includes a first electrode, a second electrode, and an organic film interposed between the first electrode and the second electrode. In one embodiment, the organic film includes the amine-based compound.

The organic layer may be a hole injection layer, a hole transport layer, or an emissive layer.

According to another aspect of the present invention, a flat panel display device is provided. The flat panel display device includes the organic light emitting device and a thin-film transistor, where the electrode of the organic light emitting device is electrically connected to a source electrode or a drain electrode of the thin-film transistor.

### BRIEF DESCRIPTION OF THE DRAWINGS

These and other features of the present invention will be better understood by reference to the following detailed description when considered in conjunction with the attached drawings in which:

FIG. 1 is a diagram illustrating a structure of an organic light emitting device according to an embodiment of the present invention; and

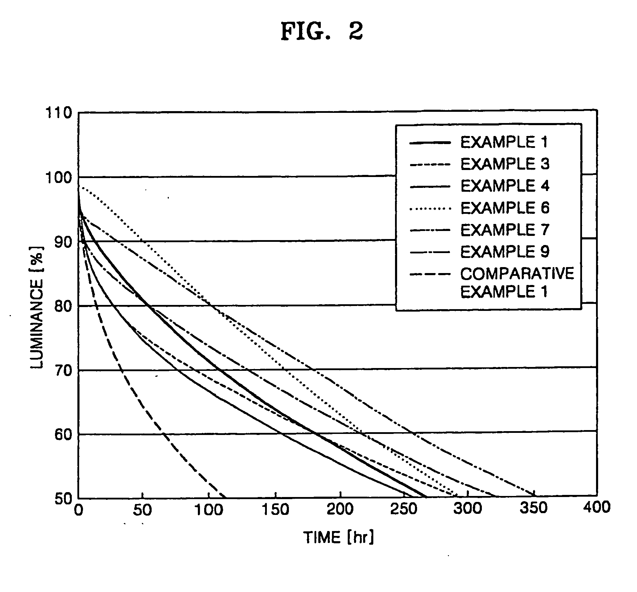

FIG. 2 is a graph comparing the life spans of an organic light emitting device according to embodiments of the present invention and a conventional organic light emitting device.

### DETAILED DESCRIPTION OF THE INVENTION

The present invention will now be described in detail with reference to the accompanying drawings, in which various embodiments of the invention are shown by way of example.

An amine-based compound represented by Formula 1 below has a carbazolyl group and a fluorenyl group.

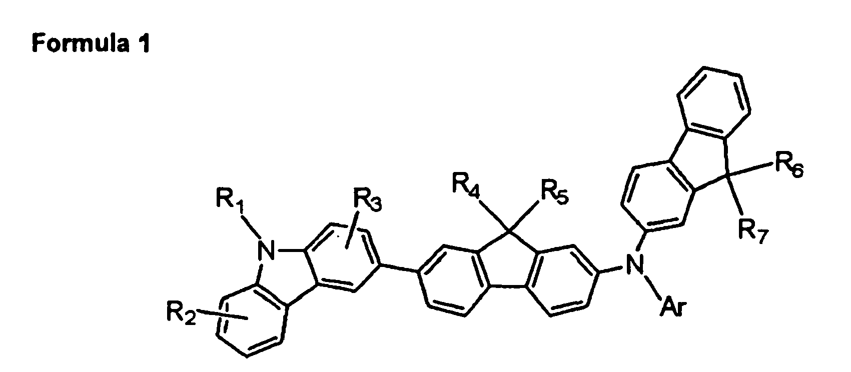

The amine-based compound of Formula 1 having both a carbazolyl group and a fluorenyl group may function as a hole injecting material, a hole transporting material, and/or a light emitting material.

The amine-based compound of Formula 1 has a high glass transition temperature (Tg) or melting point due to the presence of the carbazolyl group. As a result, it is highly resistant to Joule's heat produced inside the organic layers, between the organic layers or between an organic layer and a metal electrode. Therefore, an organic light emitting device including the amine-based compound of Formula 1 is highly durable under all kind of conditions, including manufacturing, storing, and operating conditions.

In addition, the presence of the fluorenyl group in the amine-based compound of Formula 1 further improves the film properties. As a result, organic light emitting devices with an organic film having the amine-based compound of Formula 1 have improved performance and overall film characteristics.

In Formula 1, Ar may be a substituted or unsubstituted C₆-C₃₀ aryl group, a substituted or unsubstituted C₆-C₂₀ aryloxy group, a substituted or unsubstituted C₄-C₂₀ heteroaryl group, or a substituted or unsubstituted C₄-C₂₀ condensed polycyclic group.

In particular, Ar may be a phenyl group, a biphenyl group, a terphenyl group, a naphthyl group, a pentalenyl group, an indenyl group, an anthracenyl group, an azulenyl group, a heptalenyl group, an acenaphthylenyl group, a fluorenyl group, a penanthrenyl group, a triphenylenyl group, a naphthacenyl group, a phenalenyl group, a pyrenyl group, a pentaphenyl group, a hexaphenyl group, a pyridinyl group, a quinolinyl group, a pyrazinyl group, a pyrimidinyl group, or a carbazolyl group; all of which mentioned groups may be unsubstituted or substituted with a substituent. The substituent may be selected from halogen atoms, cyano groups, amino groups, (C₁-C₁₀ alkyl)amino groups, di(C₁-C₁₀ alkyl)amino groups, (C₆-C₁₄ aryl)amino groups, di(C₆-C₁₄ aryl)amino groups, C₁-C₁₀ alkyl groups, C₁-C₁₀ alkoxy groups, C₆-C₁₄ aryloxy groups, C₆-C₁₄ aryl groups, and C₆-C₁₄ aryl groups substituted with a C₆-C₁₄ aryl substituent. However, Ar is not limited thereto.

Other suitable materials for Ar include phenyl groups, lower alkyl phenyl groups, lower alkoxy phenyl groups, cyanophenyl groups, phenoxyphenyl groups, fluorophenyl groups, naphthyl groups, lower alkyl naphthyl groups, lower alkoxy naphthyl groups, cyanonaphthyl groups, halonaphthyl groups, fluorenyl groups, carbazolyl groups, lower alkyl carbazolyl groups, biphenyl groups, lower alkyl biphenyl groups, lower alkoxy biphenyl groups, terphenyl groups, pyridyl groups, and quinolinyl groups. The lower alkyl and lower alkoxy groups may have a carbon number ranging from 1 to 5.

Other nonlimiting examples of suitable materials for Ar include phenyl groups, ethylphenyl groups, ethylbiphenyl groups, o-, m-, or p-fluorophenyl groups, dichlorophenyl groups, cyanophenyl groups, trifluorophenyl groups, methoxyphenyl groups, o-, m-, or p-tolyl groups, mesityl groups, phenoxyphenyl groups, (α, α-dimethyl benzene)phenyl groups, (N,N'-dimethyl)aminophenyl groups, (N,N'-diphenyl) aminophenyl groups, pentalenyl groups, naphthyl groups, methylnaphthyl groups, anthracenyl groups, azulenyl groups, heptalenyl groups, acenaphthylenyl groups, fluorenyl groups, anthraquinolyl groups, phenanthryl groups, triphenylene groups, naphthacenyl groups, pyrenyl groups, biphenyl groups, methylbiphenyl groups, pentaphenyl groups, hexaphenyl groups, carbazolyl groups, pyridyl groups, and quinolyl groups.

According to certain embodiments, Ar is a monocyclic, bicyclic, or tricyclic aryl group selected from phenyl groups, naphthyl groups, biphenyl groups, terphenyl groups, fluorenyl groups, and carbazolyl groups, and monocyclic, bicyclic, and tricyclic aryl groups thereof substituted with 1 to 3 substituents selected from C₁-C₅ alkyl groups, C₁-C₅ alkoxy groups, cyano groups, amino groups, phenoxy groups, phenyl groups, and halogen atoms.

According to still further embodiments, Ar is any one of the groups represented by Formulae 2a through 2q below, but is not limited thereto:

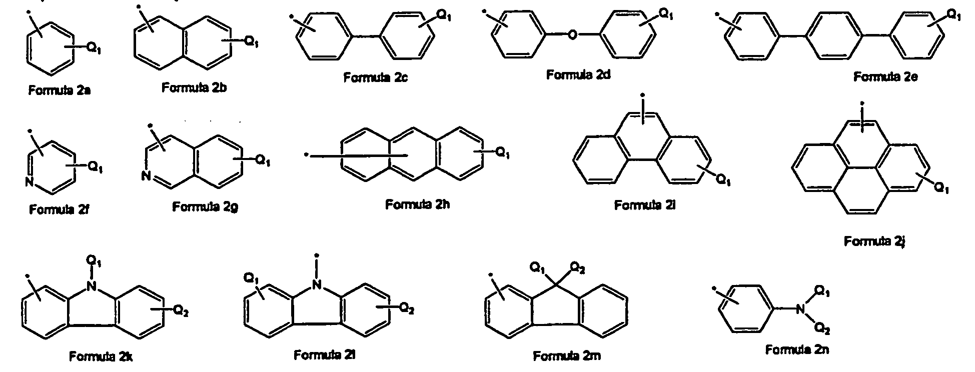

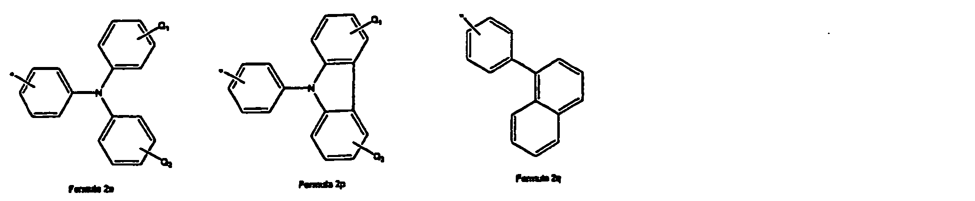

Each of Q₁ and Q₂ in Formulae 2a through 2q may independently be hydrogen, fluorine, a cyano group, an amino group, a C₁-C₁₀ alkyl group, a C₁-C₁₀ alkoxy group, a C₆-C₁₄ aryl group, or a C₆-C₁₄ aryl group substituted with a C₆-C₁₄ aryl substituent. The symbol, *, in Formulae 2a through 2q represents a binding site with N of Formula 1. The C₆-C₁₄ aryl group substituted with a C₆-C₁₄ aryl substituent may be, for example, a biphenyl group or terphenyl group substituted with a C₁-C₁₀ alkyl substituent, or a C₁-C₁₀ alkoxy group.

In certain embodiments, Ar is a group represented by Formula 2a, 2b, 2c, or 2h.

According to certain preferred embodiments, Ar is a phenyl group, a naphthyl group, a p-fluorophenyl group, a biphenyl group or a (N, N'-diphenyl) aminophenyl group.

In Formula 1, R₁ may be hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ heteroaryl group, or a substituted or unsubstituted C₄-C₂₀ condensed polycyclic group.

In particular, R₁ may be a subsituted or unsubstituted C₆-C₁₄ aryl group.

More particulary, R₁ may be a phenyl group, a naphthyl group, a pentalenyl group, an indenyl group, an anthracenyl group, an azulenyl group, a heptalenyl group, an acenaphthylenyl group, a fluorenyl group, a penanthrenyl group, a triphenylenyl group, a naphthacenyl group, a penalenyl group, a pyrenyl group, a pentaphenyl group, or a hexaphenyl group, all of which mentioned groups may be unsubstituted or substituted with a substituent. The substituent can be selected from halogen atoms, cyano groups, amino groups, (C₁-C₁₀ alkyl)amino groups, di(C₁-C₁₀ alkyl)amino groups, (C₆-C₁₄ aryl)amino groups, di(C₆-C₁₄ aryl)amino groups, C₁-C₁₀ alkyl groups, C₁-C₁₀ alkoxy groups, C₆-C₁₄ aryloxy groups, C₆-C₁₄ aryl groups, and C₆-C₁₄ aryl groups substituted with a C₆-C₁₄ aryl substituent. However, R₁ is not limited thereto.

In one embodiment, R₁ is a phenyl group, a naphthyl group, a halophenyl group, or a phenyl group substituted with a phenyl substituent (biphenyl group).

According to certain preferred embodiments, R₁ is a phenyl group, a naphthyl group, a p-fluorophenyl group or a biphenyl group.

In Formula 1, each of R₂ and R₃ may independently be hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ condensed polycyclic group, fluorine, a cyano group, or an amino group.

In particular, each of R₂ and R₃ may independently be hydrogen, fluorine, a cyano group, an amino group, a C₁-C₁₀ alkyl group, or a C₁-C₁₀ alkoxy group. More particularly, R₂ and R₃ can be both hydrogen.

In Formula 1, each of R₄, R₅, R₆, and R₇ may independently be hydrogen, a substituted or unsubstituted C₁-C₂₀ alkyl group, a substituted or unsubstituted C₁-C₂₀ alkoxy group, or a substituted or unsubstituted C₆-C₃₀ aryl group.

In particular, each of R₄ through R₇ may independently be hydrogen, a C₁-C₁₀ alkyl group, a C₁-C₁₀ alkoxy group, or a C₆-C₁₄ aryl group. More particularly, each of R₄ through R₇ may independently be hydrogen, a C₁-C₁₀ alkyl group, a phenyl group, or a naphthyl group.

The unsubstituted C₁-C₂₀ alkyl group may be linear or branched. Nonlimiting examples of suitable unsubstituted C₁-C₂₀ alkyl groups include methyl groups, ethyl groups, propyl groups, isobutyl groups, sec-butyl groups, pentyl groups, iso-amyl groups, hexyl groups, heptyl groups, octyl groups, nonyl groups, and dodecyl groups. At least one hydrogen atom of the alkyl group may be substituted with a halogen, a hydroxyl group, a nitro group, a cyano group, an amino group, an amidino group, hydrazine, hydrazone, a carboxyl group or a salt thereof, a sulfonic acid group or a shalt thereof, a phosphoric acid group or a salt thereof, a C₁-C₁₀ alkyl group, a C₁-C₁₀ alkoxy group, a C₂-C₁₀ alkenyl group, a C₂-C₁₀ alkynyl group, a C₆-C₁₆ aryl group, or a C₄-C₁₆ heteroaryl group.

The unsubstituted C₁-C₂₀ alkoxy group is a group that may have a - OA structure, where A is an unsubstituted C₁-C₂₀ alkyl group as described above. Nonlimiting examples of suitable unsubstituted C₁-C₂₀ alkoxy groups include methoxy groups, ethoxy groups, propoxy groups, isopropoxy groups, butoxy groups, and pentoxy groups. At least one hydrogen atom of the alkoxy group may be substituted with the same substituent groups as previously described for the alkyl groups.

In one embodiment, the unsubstituted C₆-C₃₀ aryl group is a C₆-C₃₀ carbocyclic aromatic system containing at least one ring. In a two-ring system, at least two rings can be fused with each other or attached to each other by single bond. In one embodiment, at least one hydrogen atom of the aryl group is substituted with the same substituent groups as previously described for the alkyl groups.

Nonlimiting examples of suitable substituted or unsubstituted C₆-C₃₀ aryl groups include phenyl groups, C₁-C₁₀ alkylphenyl groups (for example, ethyl phenyl groups), halophenyl groups (for example, o-, m- or p-fluorophenyl groups, dichlorophenyl groups), cyanophenyl groups, dicyanophenyl groups, trifluoromethoxyphenyl groups, biphenyl groups, halobiphenyl groups, cyanobiphenyl groups, C₁-C₁₀ alkyl biphenyl groups, C₁-C₁₀ alkoxybiphenyl groups, o-, m- or p-tolyl groups, o-, m- or p-cumenyl groups, mesityl groups, phenoxyphenyl groups, (a,a-dimethylbenzene)phenyl groups, (N,N'-dimethyl)aminophenyl groups, (N,N'-diphenyl)aminophenyl groups, pentalenyl groups, indenyl groups, naphthyl groups, halonaphthyl groups (for example, fluoronaphthyl groups), C₁-C₁₀ alkylnaphthyl groups (for example, methytnaphthyl groups), C₁-C₁₀ alkoxynaphthyl groups (for example, methoxynaphthyl groups), cyanonaphthyl groups, anthracenyl groups, azulenyl groups, heptalenyl groups, acenaphthylenyl groups, penalenyl groups, fluorenyl groups, anthraquinolyl groups, methylanthryl groups, phenanthryl groups, triphenylene groups, pyrenyl groups, chrysenyl groups, ethyl-chrysenyl groups, picenyl groups, perylenyl groups, chloroperylenyl groups, pentaphenyl groups, pentacenyl groups, tetraphenylenyl groups, hexaphenyl groups, hexacenyl groups, rubicenyl groups, coroneryl groups, trinaphthylenyl groups, heptaphenyl groups, heptacenyl groups, pyranetrenyl groups, and ovalenyl groups.

In one embodiment, the unsubstituted C₆-C₃₀ aryloxy group has a formula -OB, where B is a C₆-C₃₀ aryl group as described above. Nonlimiting examples of suitable C₆-C₃₀ aryloxy groups include phenoxy groups and naphthyloxy groups. At least one hydrogen atom of the aryloxy group may be substituted with the same substituent groups as previously described for the alkyl groups.

In one embodiment, the unsubstituted C₄-C₂₀ heteroaryl group includes an atom selected from the group consisting of N, O, P, and S, and at least one aromatic ring. At least two rings may be fused with each other or attached to each other by a single bond. At least one hydrogen atom of the heteroaryl group may be substituted with the same substituent groups as previously described for the alkyl groups.

Nonlimiting examples of suitable unsubstituted C₄-C₂₀ heteroaryl groups include pyrazolyl groups, imidazolyl groups, oxazolyl groups, thiazolyl groups, triazolyl groups, tetrazolyl groups, oxadiazolyl groups, pyridyl groups, pyridazinyl groups, pyrimidinyl groups, triazinyl groups, carbazolyl groups, indolyl groups, quinolyl groups, and isoquinolyl groups. These groups may be substituted with the same substituent groups as previously described for the alkyl groups.

In one embodiment, the unsubstituted C₄-C₂₀ condensed polycyclic group is a substituent including at least two rings, where at least one aromatic ring and/or at least one non-aromatic ring are fused with each other. In another embodiment, they aryl group or heteroaryl group as described above may be included in the condensed polycyclic group

In certain preferred embodiments, each of R₄, R₅, R₆ and R₇ is independently a methyl group or a phenyl group. In some preferred embodiments, both R₄ and R₅ are methyl groups or both R₄ and R₅ are phenyl groups. In some preferred embodiments both R₆ amd R₇ are methyl groups.

Nonlimiting examples of suitable amine-based compounds of Formula 1 include Compounds 1 through 96 represented by Formula 3 below.

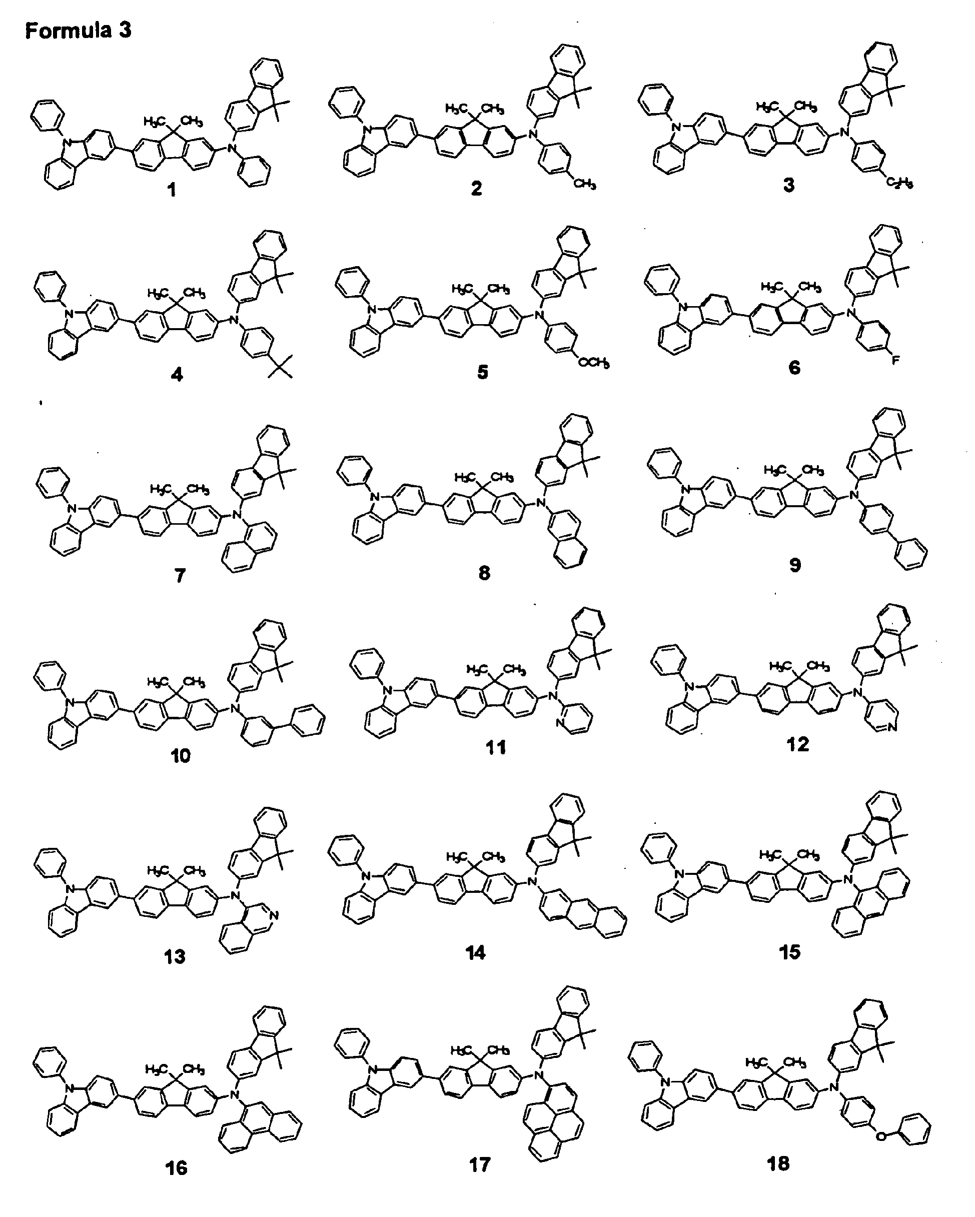

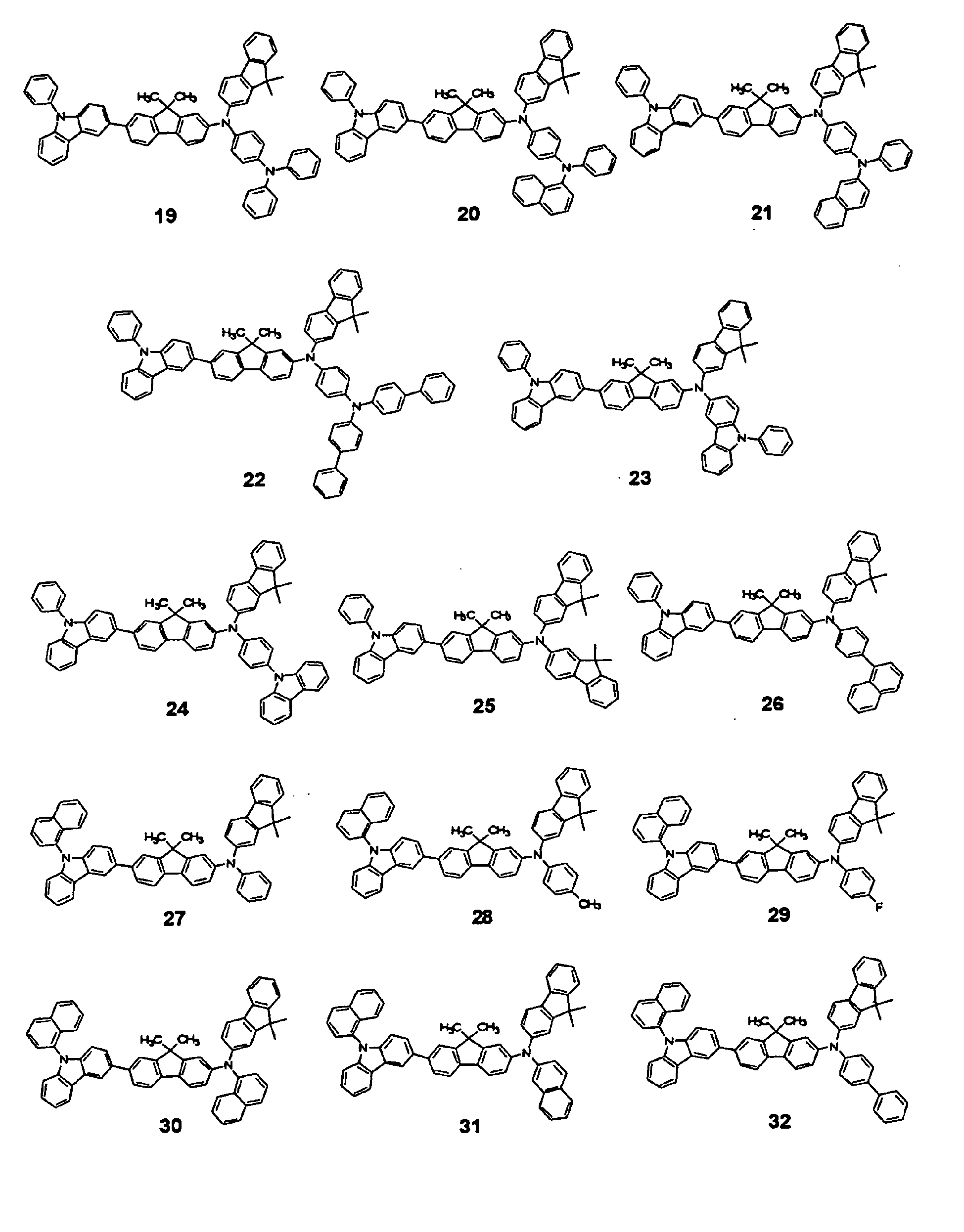

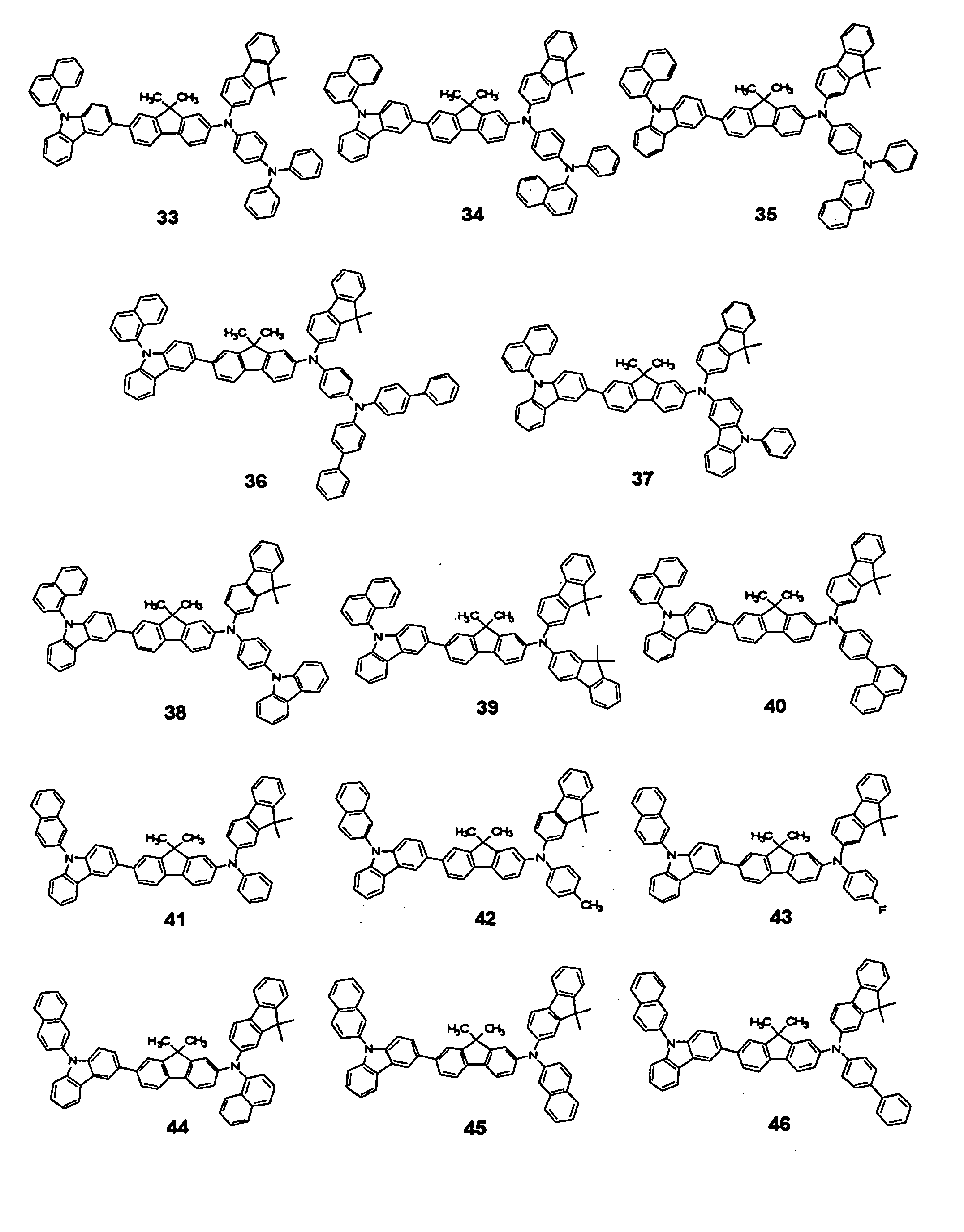

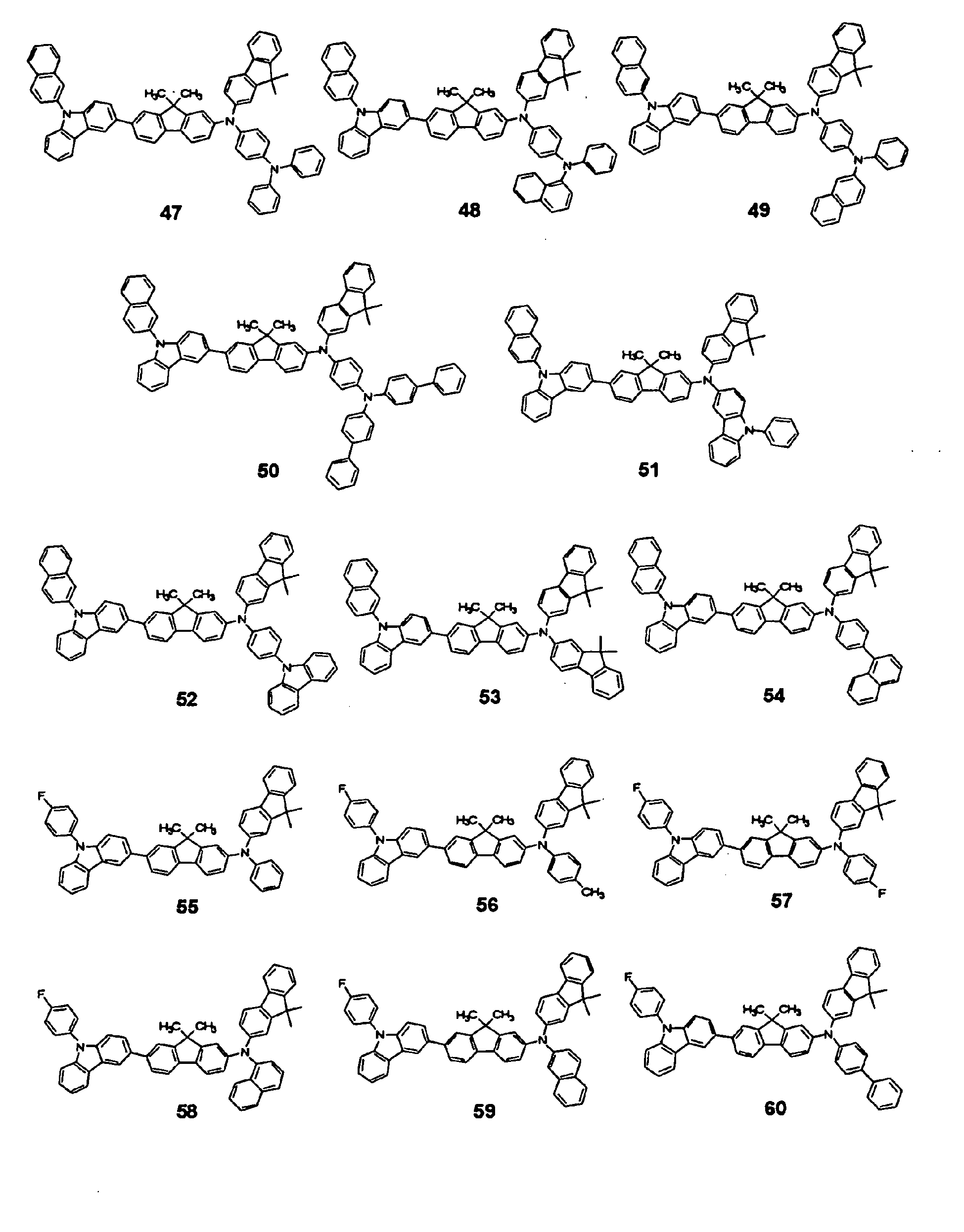

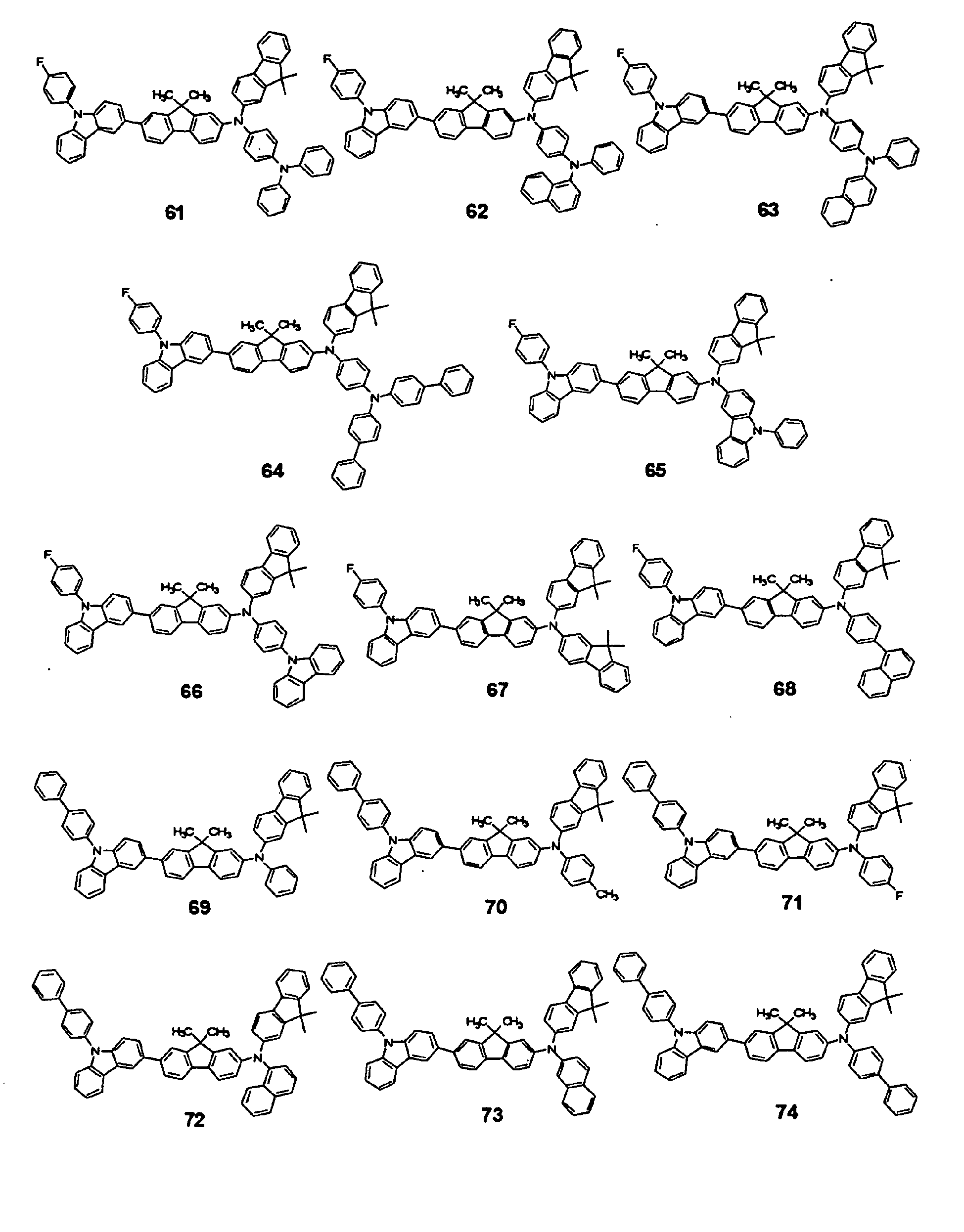

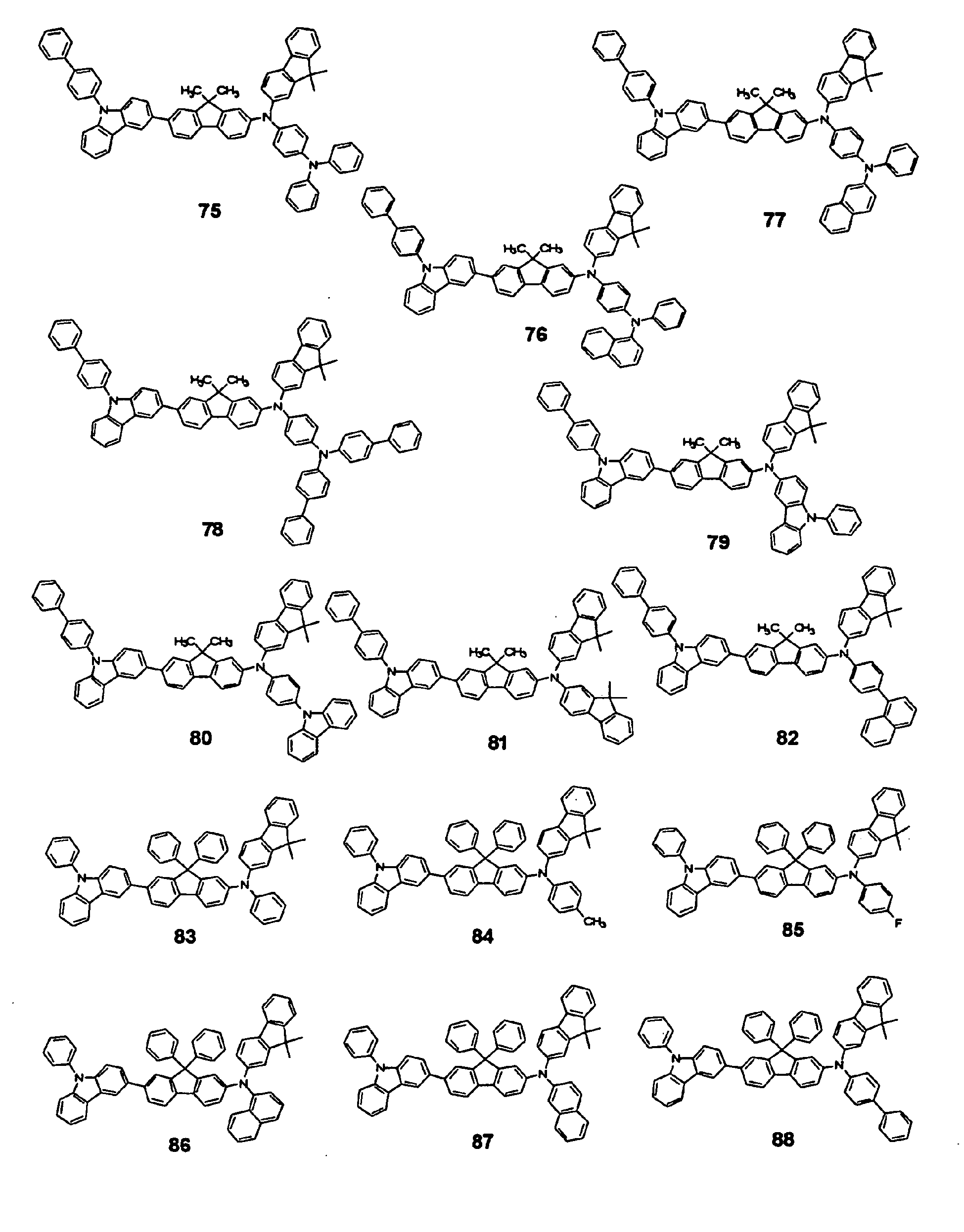

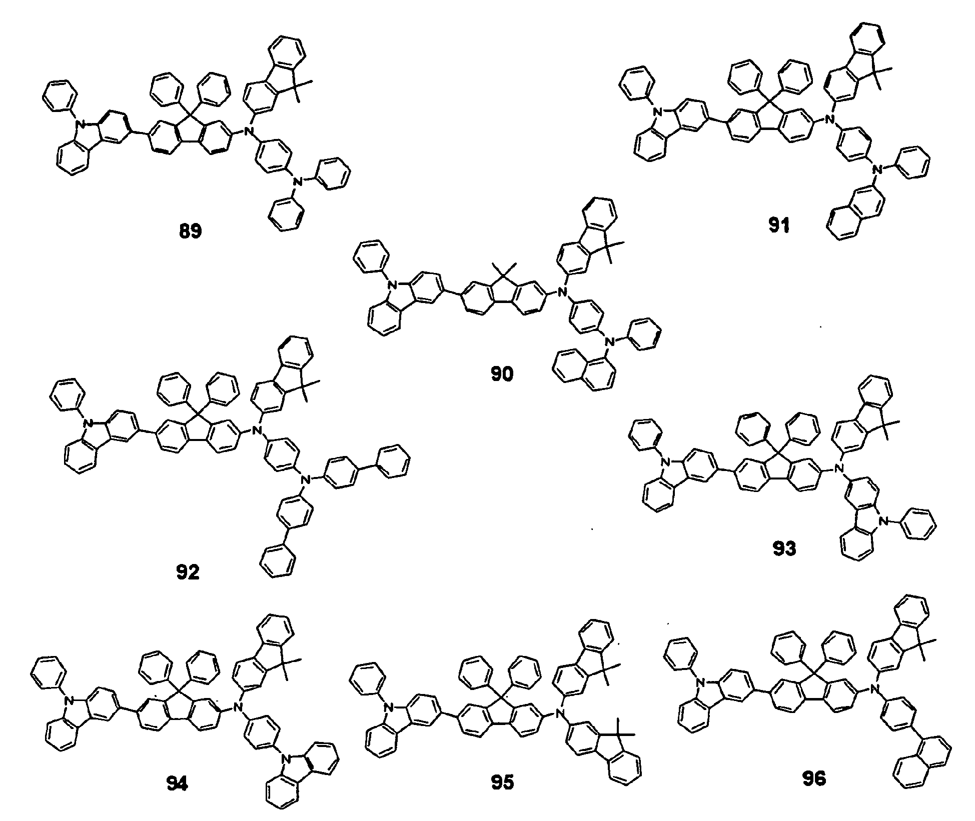

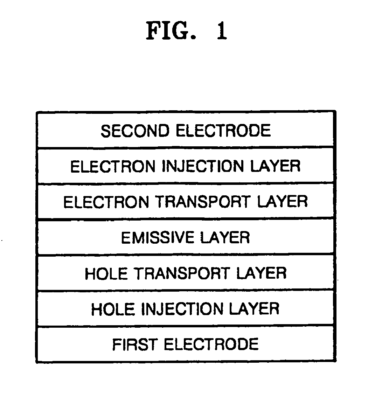

Referring now to FIG. 1, an organic light emitting device of an embodiment of the present invention includes a first electrode, a second electrode, and an organic film including the amine-based compound represented by Formula 1 between the first electrode and the second electrode. The organic film including the amine-based compound of Formula 1 may be a hole injection layer or a hole transport layer, and may also be a single film having both hole injection and hole transport capabilities. Alternatively, the organic film including the amine-based compound of Formula 1 may be an emissive layer. The amine-based compound of Formula 1 may also be used as a host material for phosphorescent or fluorescent materials of blue, green, or red colors.

In one embodiment, the organic film including the amine-based compound represented by Formula 1 is a hole transport layer.

The first electrode may be an anode, and the second electrode may be a cathode, or vice versa.

The organic light emitting device may further include a layer selected from hole injection layers, hole transport layers, electron blocking layers, emissive layers, hole blocking layers, electron transport layers, electron injection layers, and combinations thereof. In one embodiment, the organic light emitting device includes an organic layer having two layers listed above.

The organic light emitting device according to certain embodiments of the present invention may have a first electrode/hole injection layer/emissive layer/second electrode structure, a first electrode/hole injection layer/hole transport layer/emissive layer/electron transport layer/second electrode structure, or a first electrode/hole injection layer/hole transport layer/emissive layer/electron transport layer/electron injection layer/second electrode structure. Alternatively, the organic light emitting device may have a first electrode/single film with both hole injection and hole transport capabilities/emissive layer/electron transport layer/second electrode structure, or a first electrode/single film with both hole injection and hole transport capabilities/emissive layer/electron transport layer/electron injection layer/second electrode structure.

The organic light emitting device according to various embodiments of the present invention may be used in top emission type organic light emitting devices or bottom emission type organic light emitting devices.

A method of preparing the organic light-emitting device according to an embodiment of the present invention will now be described with reference to FIG. 1. The organic light-emitting device of FIG. 1 includes a substrate, a first electrode (anode), a hole injection layer, a hole transport layer, an emissive layer, an electron transport layer, an electron injection layer, and a second electrode (cathode).

First, the first electrode is formed by depositing a first electrode-forming material having a high work function on a substrate, by deposition or sputtering. The first electrode may either be an anode or a cathode. The substrate may be any substrate material conventionally used in the art. In one embodiment, the substrate is a glass substrate or a transparent plastic substrate, which has excellent mechanical strength, thermal stability, transparency, surface planarity, handling convenience, and water resistance. Nonlimiting examples of suitable first electrode-forming materials include indium tin oxide (ITO), indium zinc oxide (IZO), tin oxide (SnO₂), zinc oxide (ZnO), Al, Ag, Mg, and combinations thereof; all of which have excellent conductivity, and may be used to form a transparent or reflective electrode.

Next, the hole injection layer (HIL) may be formed on the first electrode, for example, by vacuum deposition, spin coating, casting, or Langmuir-Blodget (LB) deposition.

When the HIL is formed by vacuum deposition, the deposition conditions may vary according to the compound used, and the structure and thermal properties needed for different purposes. Generally, the deposition conditions are conducted at a deposition temperature ranging from 100 to 500°C, a vacuum pressure ranging from 10⁻⁸ to 10⁻³ torr, and at a deposition rate ranging from 0.01 to 100 Å/sec.

When the HIL is formed by spin coating, the coating conditions may vary according to the compound used, and the structure and thermal properties of the HIL needed for different purposes. Generally, the coating conditions are conducted at a coating speed ranging from about 2000 to about 5000 rpm, and a heat-treatment temperature for removing a solvent after coating ranging from 80 to 200°C.

The HIL material may be the amine-based compound represented by Formula 1 as previously described. Alternatively, other HIL materials known in the art may also be used. Nonlimiting examples of suitable HIL materials include phthalocyanine compounds such as copper phthalocyanine, 4,4',4"-tris (3-methylphenylphenylamino) triphenylamine (m-MTDATA), N,N'-di(1-naphthyl)-N,N'-diphenylbenzidine) (NPB), TDATA, 2-TNATA, polyaniline/dodecylbenzenesutfonic acid (Pani/DBSA), poly(3,4-ethylenedioxythiophene)/poly(4-styrenesulfonate (PEDOT/PSS), polyaniline/camphor sulfonicacid (Pani/CSA), and PANI/PSS (polyaniline)/poly(4-styrenesulfonate).

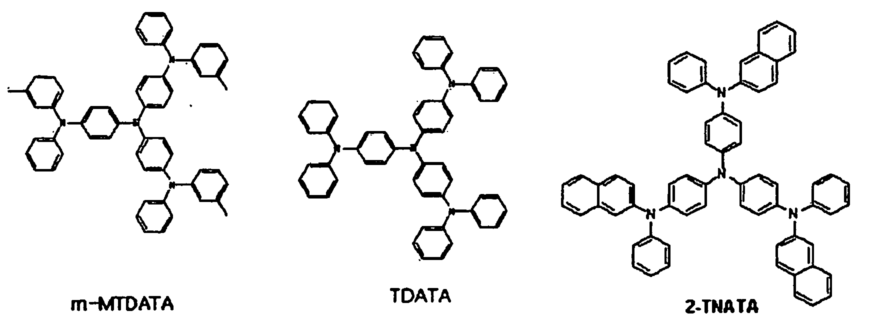

The thickness of the HIL may range from about 100 to 10000 Å, or more specifically, from 100 to 1000 Å. If the thickness of the HIL is within the above range, the HIL can have excellent hole injection properties without an increase in driving voltage. Although this range is preferred, it is possible to operate outside this range without departing from the scope of the present invention.

Next, the hole transport layer (HTL) may be formed on the HIL, for example, by vacuum deposition, spin coating, casting, or LB deposition. When the HTL is formed by vacuum deposition or spin coating, the deposition or coating conditions may vary according to the compounds used. Generally, the conditions for forming the HTL may be similar to those for forming the HIL.

The HTL material may be the amine-based compound represented by Formula 1 as previously described. Alternatively, other HTL materials known in the art may be used. Nonlimiting examples of suitable HTL materials include carbazol derivatives such as N-phenylcarbazol, polyvinylcarbazol, and amine derivatives having aromatic condensed cycles such as NPB, N,N'-bis(3-methylphenyl)- N,N'-diphenyl-[1,1-biphenyl]-4,4'-diamine (TPD), and N,N'-di(naphthalene-1-yl) -N,N'-diphenyl benzidine (a-NPD).

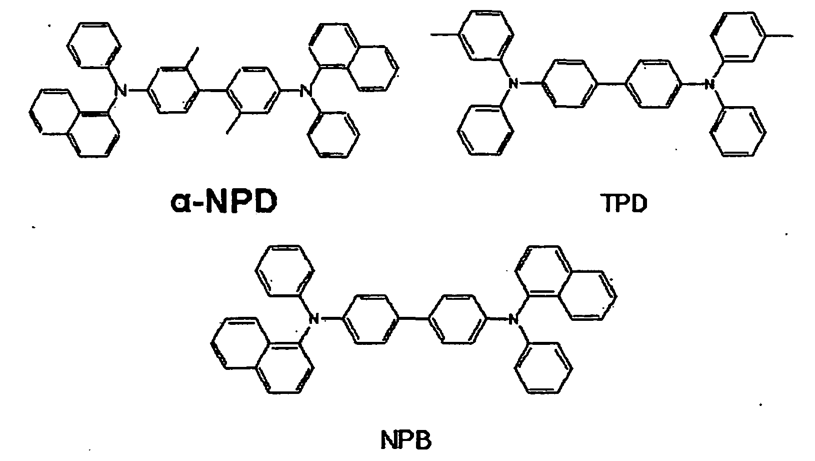

The thickness of the HTL may range from about 50 to about 1000 Å, or more specifically, from 100 to 600Å. If the thickness of the HTL is within the above range, the HTL can have excellent hole transport properties without a substantial increase in driving voltage. Although this range is preferred, it is possible to operate outside this range without departing from the scope of the present invention.

Next, the emissive layer (EML) may be formed on the HTL, for example, by vacuum deposition, spin coating, casting, or LB deposition. When the EML is formed by vacuum deposition or spin coating, the deposition or coating conditions may vary according to the compounds used, but the conditions may be similar to the conditions used to form the HIL.

The EML may include the amine-based compound represented by Formula 1 as previously described. In particular, the amine-based compound represented by Formula 1 may be used as a host or a dopant. The EML material can be any light-emitting materials, including host and dopant materials known in the art. The dopant may be either a fluorescent or a phosphorescent dopant material well known to those of ordinary skill in the art.

Nonlimiting examples of suitable host materials include Alq₃, CPB (4,4'-N,N'-dicarbazole-biphenyl), 9,10-di(naphthalene-2-yl)anthracene (ADN), and DSA (distyrylarylene).

Nonlimiting examples of suitable red dopant materials include PtOEP, Ir(piq)₃, Btp₂Ir(acac), and 4-(dicyanomethylene)-2-t-butyl-6-(1,1,7,7-tetramethyljulolidyl-9-enyl)-4H-pyran (DCJTB).

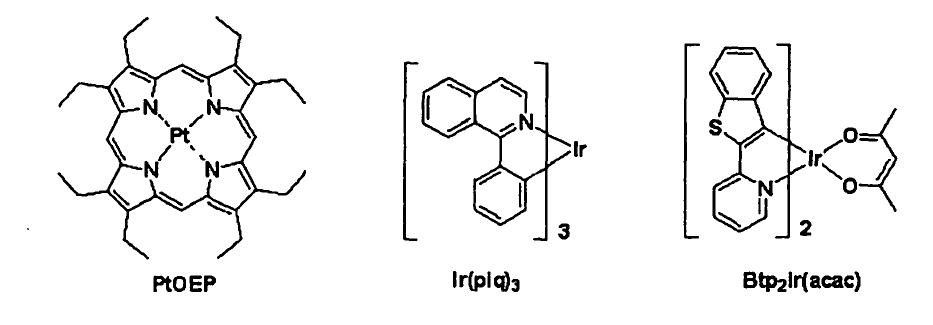

Nonlimiting examples of suitable green dopant materials include Ir(ppy)₃ (ppy = phenylpyridine), Ir(ppy)₂(acac), Ir(mpyp)₃, and C545T.

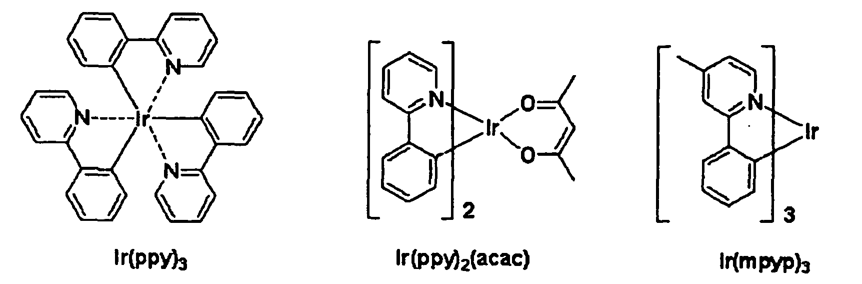

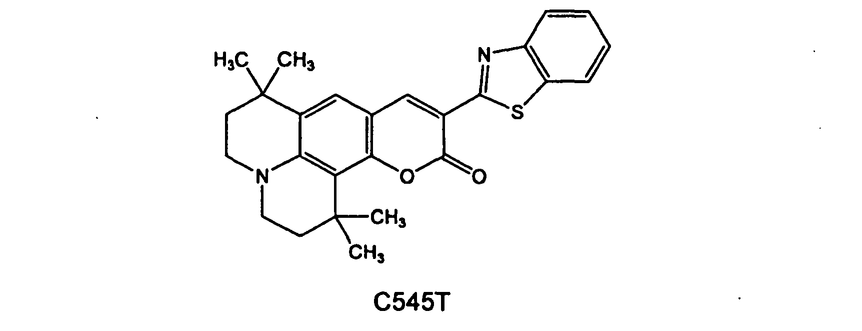

Nonlimiting examples of suitable blue dopant materials include F₂Irpic, (F₂ppy)₂Ir(tmd), Ir(dfppz)₃, ter- fluorene, 4,4'-bis(4-diphenylaminostyryl)biphenyl (DPAVBi), and 2,5,8,11-tetra-*tert*-butylperylene (TBP).

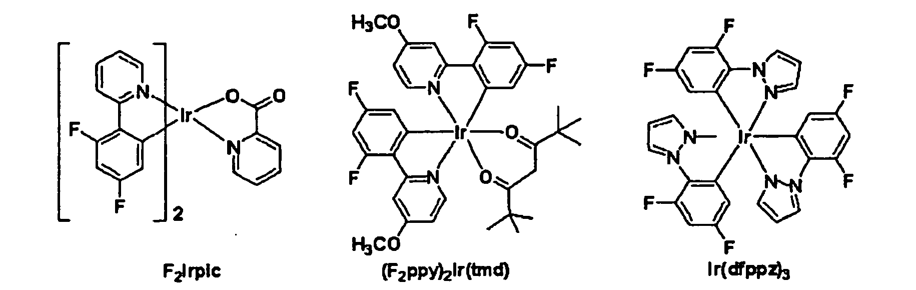

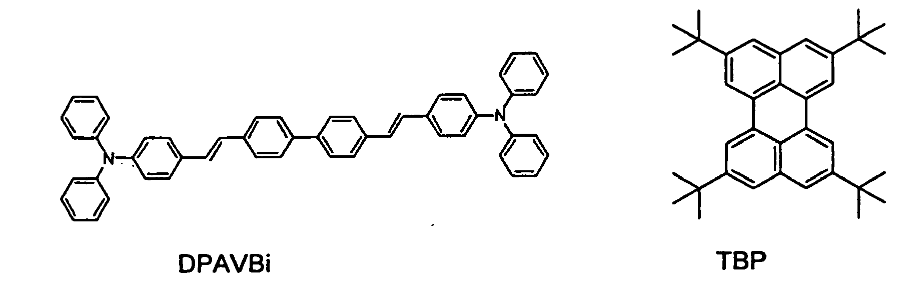

The content of the dopant may range from 0.1 to 20 parts by weight, or more specifically, from 0.5 to 12 parts by weight based on 100 parts by weight of the EML-forming material. That is, 100 parts by weight of the host and the dopant. When the content of the dopant is within the above range, concentration quenching may be substantially prevented. Although this range is preferred, it is possible to operate outside this range without departing from the scope of the present invention.

The thickness of the EML may range from 100 to 1000 Å, or more specifically, from 200 to 600 Å. If the thickness of the EML is within the above range, the EML can have excellent light-emitting properties without a substantial increase in driving voltage. Although this range is preferred, it is possible to operate outside this range without departing from the scope of the present invention.

If the EML includes a phosphorescent dopant, a hole blocking layer (HBL) may be formed on the EML to prevent triplet excitons or holes from diffusing to the electron transport layer. The HBL material can be any HBL materials known in the art. Nonlimiting examples of suitable HBL materials include oxadiazole derivatives, triazole derivatives, phenanthroline derivatives, Balq, and BCP.

The thickness of the HBL may range from 50 to 1000 Å, or more specifically, from 100 to 300 Å. If the thickness of the HBL is less than 50 Å, the hole-blocking properties may deteriorate, and if the thickness of the HBL is greater than 1000 Å, the driving voltage may increase. Although this range is preferred, it is possible to operate outside this range without departing from the scope of the present invention.

Next, the electron transport layer (ETL) may be formed on the HBL or EML, for example, by vacuum deposition, spin coating, or casting. When the ETL is formed by vacuum deposition or spin coating, the deposition or coating conditions may vary according to the compounds used. Generally, the process conditions for forming the ETL may be similar to those for forming the HIL.

The material used for the ETL can be any ETL-forming materials known in the art. Nonlimiting examples of suitable ETL materials include quinoline derivatives, tris(8-quinolinolate)aluminum (Alq₃), TAZ, and Balq.

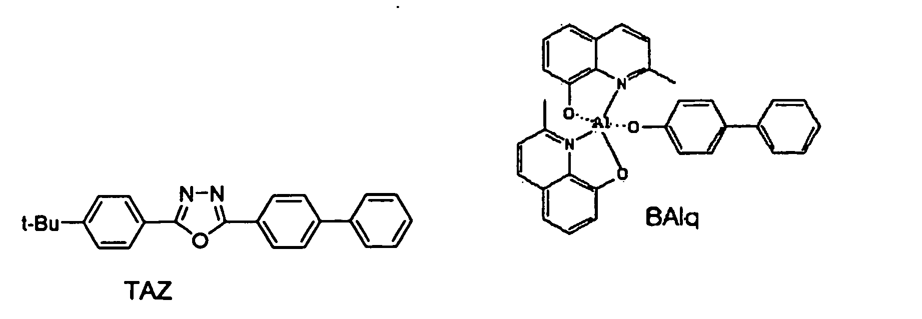

The thickness of the ETL may range from 100 to 1000 Å, or more specifically, from 100 to 500 Å. If the thickness of the ETL is within the above range, the ETL may have excellent electron transport properties without a substantial increase in driving voltage. Although this range is preferred, it is possible to operate outside this range without departing from the scope of the present invention.

In addition, an electron injection layer (EIL), which has a function of facilitating the injection of electrons from the cathode may be formed on the ETL.

Nonlimiting examples of suitable EIL materials include LiF, NaCl, CsF, Li₂O, and BaO. The deposition or coating conditions may vary according to the compounds used, but may be similar to the conditions that are used to form the HIL.

The thickness of the EIL may range from 1 to 100 Å, or more specifically, from 5 to 90 Å. If the thickness of the EIL is within the above range, the EIL may have excellent electron injection properties without a substantial increase in driving voltage. Although this range is preferred, it is possible to operate outside this range without departing from the scope of the present invention.

Finally, the second electrode may be formed on the EIL by vacuum deposition or sputtering. The second electrode may be used as a cathode or an anode. The material for forming the second electrode may be a metal, an alloy, or an electrically conductive compound with a low work function. Nonlimiting examples of such materials include Li, Mg, Al, Al-Li, Ca, Mg-In, and Mg-Ag. In addition, the second electrode may be formed of a transparent material such as ITO or IZO to provide a top-emission type organic light emitting device.

The organic light-emitting device according to various embodiments of the present invention may be used in various forms of flat-panel display devices, such as passive matrix organic light emitting display devices or active matrix organic light emitting display devices. When the organic light emitting device is included in an active matrix organic light emitting display device including a thin-film transistor, the first electrode on the substrate is a pixel electrode, which is electrically connected to a source electrode or a drain electrode of the thin-film transistor. The organic light emitting device may also be included in a flat panel display device having a double-sided screen.

The following Experimental Examples are provided for illustrative purposes only, and do not limit the scope of the present invention.

### Examples

### Synthesis Example 1: Synthesis of Intermediate 1

2.433 g of phenylcarbazole (10 mmol) was added into 100 ml of 80% acetic acid, and 1.357 g of Iodine (I₂) (5.35 mmol) and 0.333 g of ortho-periodic acid (H₂IO₆) (1.46 mmol) in a solid state were added thereto to form a mixture. The mixture was stirred at 80°C for 2 hours in a nitrogen atmosphere. A reaction was allowed to take place and terminated.

After the reaction was terminated, the mixture was extracted with 50 ml of ethylether three times. An organic layer was collected and dried using magnesium sulfate to evaporate the solvent. The residue was separately purified by silica gel column chromatography to obtain 3.23 g (yield 87%) of white solid of Intermediate 1. Intermediate 1 was evaluated by NMR, and the result thereof is shown below.
¹H NMR (CDCl₃, 300MHz) δ (ppm) 8.43 (d, 1H), 8.05 (d, 1H), 7.62 (dd, 1H), 7.61-7.75 (m, 2H), 7.51-7.43 (m, 3H), 7.41-7.35 (m, 2H), 7.27 (dd, 1H), 7.14 (d, 1H)

### Synthesis Example 2: Synthesis of Intermediate 2

Intermediate 2 was synthesized to obtain a yield of 85% in the same manner as in the synthesis of Intermediate 1, except that naphthylcarbazole was used instead of phenylcarbazole. Intermediate 2 was evaluated by NMR, and the result thereof is shown below.
¹H NMR (CDCl₃, 300MHz) δ (ppm) 8.29 (s, 1H), 8.13 (d, 1H), 7.92 (d, 1H), 7.80-7.73 (m, 3H), 7.59-7.49 (m, 5H), 7.37-7.29 (m, 3H)

### Synthesis Example 3: Synthesis of Intermediate 3

Intermediate 3 was synthesized to obtain a yield of 83% in the same manner as in the synthesis of Intermediate 1, except that 4-fluorophenylcarbazole was used instead of phenylcarbazole. Intermediate 3 was evaluated by NMR, and the result thereof is shown below.
¹H NMR (CDCl₃, 300MHz) δ (ppm) 8.30 (s, 1H), 8.13-8.11 (m, 1H), 7.74 (d, 1H), 7.56-7.52 (m, 3H), 7.37-7.29 (m, 3H), 7.08-7.03 (m, 2H)

### Synthesis Example 4: Synthesis of Intermediate 4

Intermediate 4 was synthesized to obtain a yield of 62% in the same manner as in the synthesis of Intermediate 1, except that biphenylcarbazole was used instead of phenylcarbazole. Intermediate 4 was evaluated by NMR, and the result thereof is shown below.
¹H NMR (CDCl₃, 300MHz) δ (ppm) 8.30 (s, 1H), 8.13-8.11 (m, 1H), 7.74 (d, 1H), 7.59-7.53 (m, 3H), 7.40-7.28 (m, 8H), 6.64-6.60 (m, 2H)

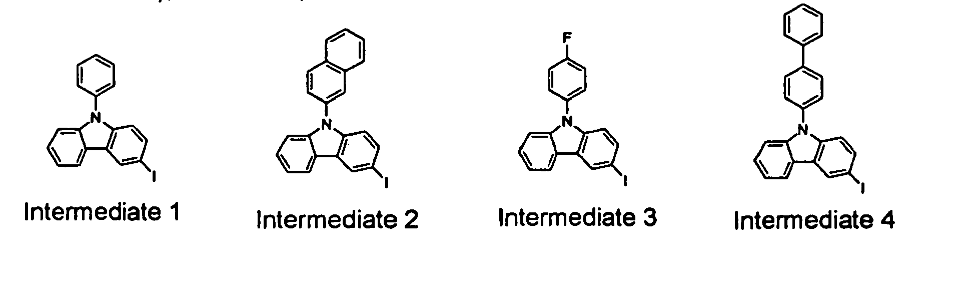

### Synthesis Example 5: Synthesis of Intermediate 5

17.6 g of 2,7-dibromo-9,9-dimethylfluorene (50 mmol) was dissolved in 150 ml of diethyl ether, and a normal butyl lithium (20 ml, 2.5 M in hexane) solution maintained at -78°C was added thereto. The temperature was raised slowly to room temperature after 30 minutes. After another 30 minutes, a solution of 23 ml (100 mmol) triisopropyl borate dissolved in 50 ml of diethyl ether maintained at -78°C was slowly added thereto to form a mixture. The mixture was stirred for 5 hours at room temperature, and water was added thereto, and the resulting mixture was washed three times with diethyl ether (200 ml). The washed diethyl ether layer was dried with MgSO₄, and was further dried under reduced pressure to obtain a product, which was recrystallized with normal hexane to obtain 10.3 g (yield: 65%) of white solid Intermediate 5. Intermediate 5 was evaluated by NMR, and the result thereof is shown below.
¹H NMR (CDCl₃, 400MHz) δ (ppm) - 8.09 (s, 2OH), 7.84-7.81 (dd, 1H), 7.75-7.72 (m, 2H), 7.67-7.65 (dd, 1H), 7.49-7.46 (dd, 1H), 7.40 (d, 1H), 1.85 (s, 6H)

### Synthesis Example 6: Synthesis of Intermediate 6

Intermediate 6 was synthesized to obtain a yield of 58% in the same manner as in the synthesis of Intermediate 5, except that 2,7-dibromo-9,9-diphenylfluorene was used instead of 2,7-dibromo-9,9-dimethylfluorene. Intermediate 6 was evaluated by NMR, and the result thereof is shown below.
¹H NMR (CDCl₃, 400MHz) δ (ppm) - 8.27 (d, 1H), 8.09 (s, 20H), 7.94-7.92 (dd, 1H), 7.81 (d, 1H), 7.74 (d, 1H), 7.69 (d, 1H), 7.60-7.51 (m, 5H), 7.18 (t, 4H), 7.02-6.97 (m, 2H)

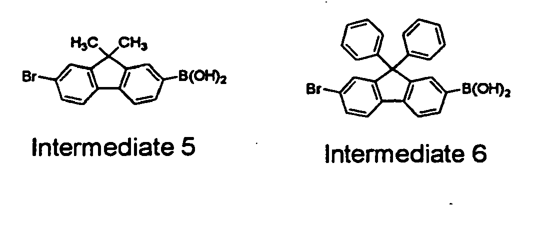

### Synthesis Example 7: Synthesis of Intermediate 7

8.2 g (30 mmol) of 2-bromo-9,9-dimethylfluorene, 4.1 mL (45 mmol) of aniline, 4.3 g (45 mmol) of t-BuONa, 0.55 g (0.6 mmol) of Pd₂(dba)₃, and 0.12 g (0.6 mmol) of P(t-Bu)₃ were dissolved in 100 ml of toluene to form a mixture. The mixture was stirred at 90°C for 3 hours. A reaction was allowed to take place and after the reaction was completed, the reaction mixture was cooled to room temperature. The mixture was extracted three times with 100 ml of distilled water and diethyl ether. An organic layer was collected and was dried using magnesium sulfate to evaporate the solvent. The residue was separately purified by silica gel column chromatography to obtain 7.87 g (yield 92%) of Intermediate 7. Intermediate 7 was evaluated by NMR, and the results thereof are shown below.
¹H NMR (CDCl₃, 400MHz) δ (ppm) - 7.82 (d, 1H), 7.54-7.49 (m, 2H), 7.27-7.21 (m, 3H), 7.12-7.08 (m, 3H), 6.97-6.93 (m, 1H), 6.90-6.86 (m, 1H), 6.59-6.56 (m, 1H), 5.44(NH), 1.85(s, 6H)
¹³C NMR (CDCl₃, 100MHz) δ (ppm) - 149.1, 146.8, 142.2, 140.0, 134.6, 133.5, 129.4, 127.8, 127.1, 126.8, 120.4, 119.7, 119.1, 117.4, 109.0, 107.5, 44.9, 24.5

### Synthesis Example 8: Synthesis of Intermediate 8

Intermediate 8 was synthesized to obtain a yield of 78% in the same manner as in the synthesis of Intermediate 7, except that 4-fluoroaniline was used instead of aniline. Intermediate 8 was evaluated by NMR, and the results thereof are shown below.
¹H NMR (CDCl₃, 400MHz) δ (ppm) - 7.95-7.91 (m, 2H), 7.82 (d, 1H), 7.54-7.49 (m, 2H), 7.24-7.19 (m, 3H), 7.12 (d, 1H), 6.97-6.93 (m, 1H), 6.59-6.56 (dd, 1H), 5.44(s, NH), 1.85(s, 6H)
¹³C NMR (CDCl₃, 100MHz) δ (ppm) - 161.0, 154.5, 149.1, 146.8, 140.0, 138.3, 134.6, 132.5, 127.8, 127.1, 126.8, 120.4, 119.7, 116.5, 115.8, 112.3, 112.1, 109.6, 107.5, 44.9, 24.5

### Synthesis Example 9: Synthesis of Intermediate 9

Intermediate 9 was synthesized to obtain a yield of 83% in the same manner as in the synthesis of Intermediate 7, except that 1-bromonaphthalene was used instead of aniline. Intermediate 9 was evaluated by NMR, and the results thereof are shown below.
¹H NMR (CDCl₃, 400MHz) δ (ppm) - 8.41 (dd, 1H), 7.82 (d, 1H), 7.72 (d, 1H), 7.59-7.36 (m, 5H), 7.24-7.09 (m, 4H), 6.97-6.93 (m, 1H), 6.55 (dd, 1H), 5.83 (s, NH), 1.85(s, 6H)
¹³C NMR (CDCl₃, 100MHz) δ (ppm) - 149.1, 148.7, 144.3, 140.0, 137.2, 134.6, 133.3, 128.8, 128.2, 127.8, 127.1, 126.8, 125.0, 124.5, 122.4, 122.3, 121.3, 119.7, 116.8, 109.2, 107.7, 99.0, 44.9, 24.5

### Synthesis Example 10: Synthesis of Intermediate 10

Intermediate 10 was synthesized to obtain a yield of 85% in the same manner as in the synthesis of Intermediate 7, except that 4-aminobiphenyl was used instead of aniline. Intermediate 10 was evaluated by NMR, and the results thereof are shown below.
¹H NMR (CDCl₃, 400MHz) δ (ppm) - 7.82 (d, 1H), 7.60-7.49 (m, 6H), 7.40-7.29 (m, 3H), 7.24-7.20 (m, 1H), 7.12 (d, 1H), 6.95 (t, 1H), 6.79-6.75 (m, 2H), 6.59-6.56 (m, 1H), 5.44 (s, NH), 1.85 (s, 6H)
¹³C NMR (CDCl₃, 100MHz) δ (ppm) - 149.1, 146.8, 140.0, 138.8, 137.4, 134.9, 134.6, 132.9, 129.9, 128.8, 127.8, 127.2, 127.1, 126.8, 120.4, 119.7, 116.3, 109.0, 107.5, 44.9, 24.5

### Synthesis Example 11: Synthesis of Intermediate 11

Intermediate 11 was synthesized to obtain a yield of 74% in the same manner as in the synthesis of Intermediate 7, except that N,N'-dibiphenyl-1,4-phenylenediamine was used instead of aniline. Intermediate 11 was evaluated by NMR, and the results thereof are shown below.
¹H NMR (CDCl₃, 400MHz) δ (ppm) - 7.82 (d, 1H), 7.76-7.64 (m, 8H), 7.54-7.49 (m, 2H), 7.40-7.11 (m, 10H), 6.97-6.93 (m, 1H), 6.60-6.43 (m, 7H), 5.44 (s, NH), 1.85(s, 6H)
¹³C NMR (CDCl₃, 100MHz) δ (ppm) - 149.1, 147.9, 146.8, 143.8, 140.0, 136.9, 136.4, 135.4, 134.6, 134.2, 132.3, 128.9, 128.8. 127.8, 127.2, 127.1, 126.8, 120.4, 119.7, 117.7, 114.6, 109.0, 107.5, 44.9, 24.5

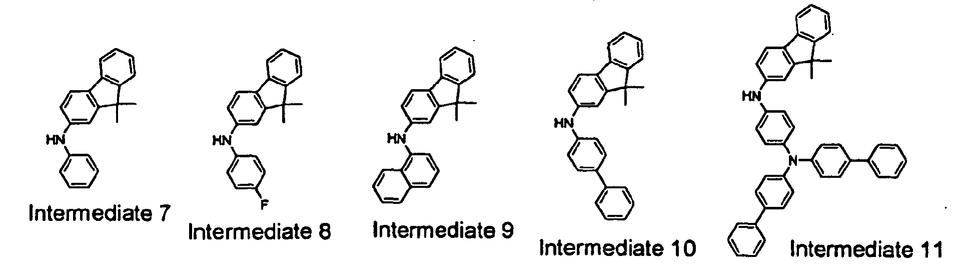

### Synthesis Example 12: Synthesis of Intermediate 12

9.51 g (30 mmol) of Intermediate 5, 16.6 g (60 mmol) of Intermediate 1, 1.7 g (1.5 mmol) of Pd(PPh₃)₄, and 20 g (150 mmol) of K₂CO₃ were dissolved in 100 ml of a mixed solution of THF/H₂O (2:1) to form a mixture. The mixture was stirred at 80°C for 5 hours, and a reaction was allowed to take place. The reaction mixture was extracted with 600 ml of diethyl ether three times. An organic layer was collected and dried using magnesium sulfate, and the residue obtained by evaporating the solvent was recrystallized with dichloromethane and normal hexane to obtain 10.03 g (yield 65%) of Intermediate 12. Intermediate 12 was evaluated by NMR, and the results thereof are shown below.
¹H NMR (CDCl₃, 400MHz) δ (ppm) - 8.16-8.14 (m, 1H), 7.94 (d, 1H), 7.68 (d, 1H), 7.63 (s, 1H), 7.50-7.46 (m, 5H), 7.36-7.27 (m, 5H), 7.07-6.94 (m, 4H), 1.85 (s, 6H)
¹³C NMR (CDCl₃, 100MHz) δ (ppm) - 163.3, 150.7, 144.7, 144.2, 141.6, 141.0, 137.0, 136.0, 134.0, 131.8, 131.6, 129.8, 129.3, 129.2, 127.4, 127.1, 126.8, 126.3, 121.8, 121.2, 120.6, 120.4, 119.9, 119.5, 118.6, 115.4, 114.5, 109.0, 49.6, 24.5

### Synthesis Example 13: Synthesis of Intermediate 13

Intermediate 13 was synthesized to obtain a yield of 63% in the same manner as in the synthesis of Intermediate 12, except that Intermediate 2 was used instead of Intermediate 1. Intermediate 13 was evaluated by NMR, and the results thereof are shown below.
¹H NMR (CDCl₃, 400MHz) δ (ppm) - 8.15 (d, 1H), 7.95-7.91 (m, 2H), 7.81-7.46 (m, 9H), 7.36-7.27 (m, 4H), 7.06 (d, 1H), 7.00-6.94 (m, 4H), 1.85 (s, 6H)
¹³C NMR (CDCl₃, 100MHz) δ (ppm) - 163.3, 150.7, 144.9, 144.2, 141.6, 140.0, 136.1, 136.0, 134.0, 131.8, 131.6, 129.5, 129.3, 129.2, 128.2, 127.9, 127.6, 126.8, 126.7, 126.2, 124.3, 122.5, 122.3, 121.8, 121.4, 121.2, 120.6, 119.9, 118.6, 117.3, 116.6, 114.5, 110.2, 105.1, 49.6, 24.5

### Synthesis Example 14: Synthesis of Intermediate 14

Intermediate 14 was synthesized to obtain a yield of 62% in the same manner as in the synthesis of Intermediate 12, except that Intermediate 3 was used instead of Intermediate 1. Intermediate 14 was evaluated by NMR, and the results thereof are shown below.
¹H NMR (CDCl₃, 400MHz) δ (ppm) - 8.16-8.14 (m, 1H), 7.95 (d, 1H), 7.69 (d, 1H), 7.63 (s, 1H), 7.56-7.46 (m, 3H), 7.36-7.27 (m, 4H), 7.08-6.94 (m, 6H), 1.85 (s, 6H)
¹³C NMR (CDCl₃, 100MHz) δ (ppm) - 163.3, 162.0, 155.5, 150.7, 144.2, 143.8, 141.6, 140.0, 136.1, 136.0, 134.0, 131.6, 129.3, 129.2, 126.8, 126.3, 125.5, 125.3, 121.8, 121.2, 120.6, 120.4, 119.9, 119.5, 118.6, 115.4, 114.5, 113.8, 113.1, 109.0, 49.6, 24.5

### Synthesis Example 15: Synthesis of Intermediate 15

Intermediate 15 was synthesized to obtain a yield of 58% in the same manner as in the synthesis of Intermediate 12, except that Intermediate 4 was used instead of Intermediate 1. Intermediate 15 was evaluated by NMR, and the results thereof are shown below.
¹H NMR (CDCl₃, 400MHz) δ (ppm) - 8.16-8.14 (m, 1H), 7.95 (d, 1H), 7.69 (d, 1H), 7.63 (s, 1H), 7.60-7.56 (m, 2H), 7.49-7.45 (m, 1H), 7.41-7.27 (m, 9H), 7.07-6.94 (m, 4H), 6.64-6.60 (m, 2H), 1.85 (s, 6H)
¹³C NMR (CDCl₃, 100MHz) δ (ppm)- 163.3, 150.7, 144.2, 144.1, 141.6, 140.3, 139.3, 136.9, 136.0, 135.4, 134.0, 131.9, 131.8, 131.6, 129.3, 129.2, 128.9, 128.8, 127.2, 126.8, 126.3, 121.8, 121.2, 120.6, 120.4, 119.9, 119.5, 118.6, 116.8, 115.4, 114.5, 109.0, 49.6, 24.5

### Synthesis Example 16: Synthesis of Intermediate 16

Intermediate 16 was synthesized to obtain a yield of 55% in the same manner as in the synthesis of Intermediate 12, except that Intermediate 6 was used instead of Intermediate 5. Intermediate 16 was evaluated by NMR, and the result thereof is shown below.
¹H NMR (CDCl₃, 400MHz) δ (ppm) - 8.16-8.14 (m, 1H), 7.95 (d, 1H), 7.77 (d, 1H), 7.65-7.63 (m, 2H), 7.59 (dd, 1H), 7.52-7.46 (m, 9H), 7.37-7.27 (m,
4H), 7.20-7.13 (m, 5H), 7.06 (dd, 1H), 7.02-6.97 (m, 3H) ¹³C NMR (CDCl₃, 100MHz) δ (ppm) -164.2, 151.5, 145.4, 144.7, 142.9, 141.0, 140.7, 137.0, 135.7, 135.1, 132.8, 131.3, 130.1, 129.8, 128.8, 127.8, 127.4, 127.1, 126.3, 126.0, 125.1, 122.9, 122.3, 121.7, 120.4, 119.9, 119.5, 118.6, 115.4, 114.1, 109.0, 72.9

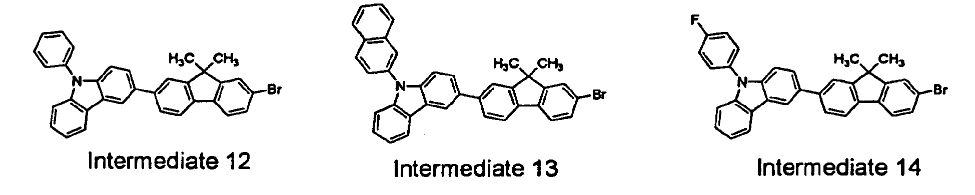

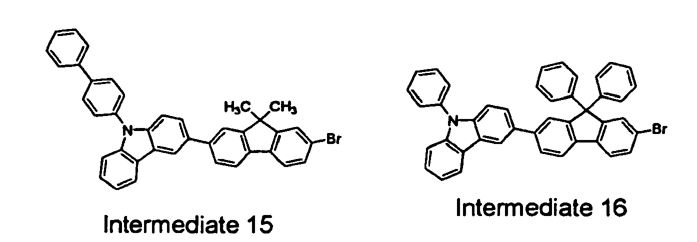

### Synthesis Example 17: Synthesis of Compound 1

5.14 g (10 mmol) of Intermediate 12, 3.42 g (12 mmol) of Intermediate 7, 2.9 g (30 mmol) of t-BuONa, 366 mg (0.4 mmol) of Pd₂(dba)₃, and 80 mg (0.4 mmol) of P(t-Bu)₃ were dissolved in 60 ml of toluene to form a mixture. The mixture was stirred at 90°C for 3 hours, and a reaction was allowed to take place. After the reaction was completed, the reaction mixture was cooled to room temperature, and extracted with distilled water and 50 ml of diethyl ether three times. An organic layer was collected and dried using magnesium sulfate, and the residue obtained by evaporating the solvent was separately purified by silica gel column chromatography to obtain 5.9 g (yield 82%) of Compound 1. Compound 1 was evaluated by NMR, and the results thereof are shown below.
¹H NMR (CDCl₃, 400MHz) δ (ppm)- 8.16-8.14 (m, 1H), 7.99-7.93 (m, 2H), 7.69-7.46 (m, 8H), 7.37-7.27 (m, 6H), 7.24-6.93 (m, 8H), 6.64-6.59 (m, 1H), 6.31 (dd, 2H), 5.65 (dd, 2H), 1.85 (s, 12H)
¹³C NMR (CDCl₃, 100MHz) δ (ppm) - 158.7,148.6,148.1,147.1,147.0, 144.9, 144.7, 141.0, 139.6, 138.0, 137.0, 136.5, 134.8, 134.0, 131.8, 129.8, 129.4, 129.3, 127.8, 127.4, 127.1, 126.8, 126.3, 126.1, 124.0, 122.9, 121.7, 121.1, 120.5, 120.4, 119.9, 119.5, 118.7, 118.6, 117.8, 117.2, 115.4, 114.5, 112.7, 109.0, 46.1, 44.9, 24.5

### Synthesis Example 18: Synthesis of Compound 6

Compound 6 was synthesized to obtain a yield of 75% in the same manner as in the synthesis of Compound 1, except that Intermediate 8 was used instead of Intermediate 7. Compound 6 was evaluated by NMR, and the results thereof are shown below.
¹H NMR (CDCl₃, 400MHz) δ (ppm) - 8.16-8.14 (m, 1H), 7.99-7.93 (m, 2H), 7.69-7.46 (m, 8H), 7.37-6.93 (m, 14H), 6.73 (dd, 2H), 6.31 (dd, 2H), 1.85(s, 12H)
¹³C NMR (CDCl₃, 100MHz) δ (ppm) - 162.0, 158.7, 155.5, 148.1, 147.1, 146.0, 144.7, 141.0, 140.7, 139.6, 138.0, 137.0, 136.5, 134.8, 134.0, 131.8, 129.8, 129.3, 127.8, 127.4, 127.1, 126.8, 126.4, 126.3, 126.2, 126.1, 121.7, 121.1, 120.5, 120.4, 119.9, 119.5, 118.7, 118.6, 117.8, 117.2, 116.2, 115.5, 115.4, 114.5, 112.7, 109.0, 46.1, 44.9, 24.5

### Synthesis Example 19: Synthesis of Compound 7

Compound 7 was synthesized to obtain a yield of 80% in the same manner as in the synthesis of Compound 1, except that Intermediate 9 was used instead of Intermediate 7. Compound 7 was evaluated by NMR, and the results thereof are shown below.
¹H NMR (CDCl₃, 400MHz) δ (ppm) - 8.50 (dd, 1H), 8.16-8.14 (m, 1H), 7.99-7.93 (m, 2H), 7.85 (dd, 1H), 7.69-6.93 (m, 24H), 6.29 (dd, 2H), 6.08 (dd, 1H), 1.85(s, 12H)
¹³C NMR (CDCl₃, 100MHz) δ (ppm) - 158.7, 150.6, 150.5, 150.0, 147.1, 144.7, 141.0, 139.6, 138.0, 137.0, 136.5, 134.8, 134.0, 131.8, 129.8, 129.3, 128.6, 127.8, 127.6, 127.4, 127.1, 126.8, 126.7, 126.3, 126.1, 126.0, 124.1, 123.5, 122.9, 121.7, 121.5, 120.5, 120.4, 119.9, 119.5, 118.7, 118.6, 118.0, 117.4, 116.4, 115.4, 114.5, 112.9, 109.0, 46.1, 44.9, 24.5

### Synthesis Example 20: Synthesis of Compound 9

Compound 9 was synthesized to obtain a yield of 77% in the same manner as in the synthesis of Compound 1, except that Intermediate 10 was used instead of Intermediate 7. Compound 9 was evaluated by NMR, and the results thereof are shown below.
¹H NMR (CDCl₃, 400MHz) δ (ppm) - 8.16-8.14 (m, 1H), 7.99-7.93 (m, 2H), 7.76-6.93 (m, 27H), 6.43-6.29 (m, 4H), 1.85 (s, 12H)
¹³C NMR (CDCl₃, 100MHz) δ (ppm) - 158.7, 148.6, 148.1, 147.1, 146.3, 145.5, 144.7, 141.0, 139.6, 138.0, 137.0, 136.9, 136.5, 135.4, 134.8, 134.2, 134.0, 131.8, 129.8, 129.3, 128.9, 128.8, 127.7, 127.4, 127.2, 127.1, 126.8, 126.3, 126.1, 117.8, 117.7, 117.2, 115.4, 114.5, 112.7, 109.0, 46.1, 44.9, 24.5

### Synthesis Example 21: Synthesis of Compound 22

Compound 22 was synthesized to obtain a yield of 72% in the same manner as in the synthesis of Compound 1, except that Intermediate 11 was used instead of Intermediate 7. Compound 22 was evaluated by NMR, and the results thereof are shown below.
¹H NMR (CDCl₃, 400MHz) δ (ppm) - 8.16-8.14 (m, 1H), 7.99-7.93 (m, 2H), 7.76-7.63 (m, 10H), 7.56 (dd, 2H), 7.49 (dd, 4H), 7.41-7.07 (m, 15H), 7.00-6.93 (m, 3H), 6.47-6.43 (m, 4H), 6.32-6.19 (m, 6H), 1.85 (s, 12H)
¹³C NMR (CDCl₃, 100MHz) δ (ppm) - 158.7, 148.6, 148.1, 147.1, 146.8, 145.7, 144.7, 143.8, 141.0, 139.6, 138.0, 137.0, 136.9, 136.5, 135.4, 134.8, 134.2, 134.0, 131.8, 129.8, 129.3, 128.9, 128.8, 127.8, 127.4, 127.2, 127.1, 126.8, 126.3, 126.1, 121.7, 121.1, 120.5, 119.9, 119.7, 119.6, 118.7, 117.8, 117.7, 117.2, 115.4, 114.5, 112.7, 109.0, 46.1, 44.9, 24.5

### Synthesis Example 22: Synthesis of Compound 46

Compound 46 was synthesized to obtain a yield of 75% in the same manner as in the synthesis of Compound 9, except that Intermediate 13 was used instead of Intermediate 12. Compound 46 was evaluated by NMR, and the results thereof are shown below.
¹H NMR (CDCl₃, 400MHz) δ (ppm) - 8.15 (d, 1H), 7.99-7.91 (m, 3H), 7.80-7.49 (m, 13H), 7.41-7.07 (m, 11H), 7.00-6.93 (m, 3H), 6.43-6.39 (m, 2H), 6.31 (dd, 2H), 1.85(s, 12H)
¹³C NMR (CDCl₃, 100MHz) δ (ppm) - 158.7, 148.6, 148.1, 147.1, 146.3, 145.5, 144.9, 140.0, 139.6, 138.0, 137.0, 136.9, 136.5, 136.1, 135.4, 134.8, 134.2, 134.0, 131.8, 129.5, 129.3, 128.9, 128.8, 128.2, 127.8, 127.6, 127.2, 126.8, 126.7, 126.2, 126.1, 124.3, 122.5, 122.3, 121.7, 121.4, 121.1, 120.5, 119.9, 118.7, 118.6, 117.8, 117.7, 117.3, 117.2, 116.6, 114.5, 112.7, 110.2, 105.1, 46.1, 44.9, 24. 5

### Synthesis Example 23: Synthesis of Compound 60

Compound 60 was synthesized to obtain a yield of 78% in the same manner as in the synthesis of Compound 9, except that Intermediate 14 was used instead of Intermediate 12. Compound 60 was evaluated by NMR, and the results thereof are shown below.
¹H NMR (CDCl₃, 400MHz) δ (ppm) - 8.16-8.14 (m, 1H), 7.99-7.93 (m, 2H), 7.76-7.52 (m, 10H), 7.41-6.93 (m, 16H), 6.43-6.39 (m, 2H), 6.31 (dd, 2H), 1.85(s, 12H)
¹³C NMR (CDCl₃, 100MHz) δ (ppm) - 161.9,158.7,155.5,148.6,148.1, 147.1, 146.3, 145.5, 143.8, 140.0, 139.6, 138.0, 137.0, 136.9, 136.5, 136.1, 135.4, 134.8, 134.2, 134.0, 131.8, 129.3, 128.9, 128.8, 127.8, 127.2, 126.8, 126.3, 126.1, 125.5, 125.3, 121.7, 121.1, 120.5, 120.4, 119.9, 119.5, 118.7, 118.6, 117.8, 117.7, 117.2, 115.4, 114.5, 113.8, 113.1, 112.7, 109.0, 46.1, 44.9, 24.5

### Synthesis Example 24: Synthesis of Compound 72

Compound 72 was synthesized to obtain a yield of 81% in the same manner as in the synthesis of Compound 7, except that Intermediate 15 was used instead of Intermediate 12. Compound 72 was evaluated by NMR, and the results thereof are shown below.
¹H NMR (CDCl₃, 400MHz) δ (ppm) - 8.50 (dd, 1H), 8.16-8.14 (m, 1H), 7.99-7.93 (m, 2H), 7.85 (dd, 1H), 7.69-6.93 (m, 26H), 6.64-6.60 (m, 2H), 6.29 (dd, 2H), 6.09 (dd, 1H), 1.85 (s, 12H)
¹³C NMR (CDCl₃, 100MHz) δ (ppm) - 158.7,150.6,150.5,150.0,147.1, 144.1, 140.3, 139.6, 139.3, 138.0, 137.0, 136.9, 136.5, 135.4, 134.8, 134.0, 131.8, 129.3, 128.9, 128.8, 128.6, 127.8, 127.6, 127.2, 126.8, 126.7, 126.3, 126.1, 126.0, 124.1, 123.5, 122.9, 121.7, 121.5, 120.5, 120.4, 119.9, 119.5, 118.7, 109.0, 46.1, 44.9, 24.5

### Synthesis Example 25: Synthesis of Compound 74

Compound 74 was synthesized to obtain a yield of 83% in the same manner as in the synthesis of Compound 9, except that Intermediate 15 was used instead of Intermediate 12. Compound 74 was evaluated by NMR, and the results thereof are shown below.
¹H NMR (CDCl₃, 400MHz) δ (ppm) - 8.16-8.14 (m, 1H), 7.99-7.93 (m, 2H), 7.76-7.53 (m, 10H), 7.41-6.93 (m, 18H), 6.64-6.60 (m, 2H), 6.43-6.39 (m, 2H), 6.31 (dd, 2H), 1.85 (s, 12H)
¹³C NMR (CDCl₃, 100MHz) δ (ppm) - 158.7,148.6,148.1,147.1,146.3, 145.5, 144.1, 140.3, 139.6, 139.3, 138.0, 137.0, 136.9, 136.5, 135.4, 134.8, 134.2, 134.0, 131.8, 129.3, 128.9, 128.8, 127.8, 127.2, 126.8, 126.3, 126.1, 121.7, 121.1, 120.5, 119.9, 119.5, 118.7, 118.6, 117.8, 117.7, 117.2, 116.8, 115.4, 114.5, 112.7, 109.0, 46.1, 44.9, 24.5

### Synthesis Example 26: Synthesis of Compound 83

Compound 83 was synthesized to obtain a yield of 69% in the same manner as in the synthesis of Compound 1, except that Intermediate 16 was used instead of Intermediate 12. Compound 83 was evaluated by NMR, and the results thereof are shown below.
¹H NMR (CDCl₃, 400MHz) δ (ppm) - 8.16-8.14 (m, 1H), 7.99-7.93 (m, 2H), 7.69-7.56 (m, 10H), 7.49 (d, 4H), 7.37-6.93 (m, 18H), 6.64-6.59 (m, 1H), 6.42 (dd, 1H), 6.31 (dd, 1H), 5.67-5.63 (m, 2H), 1.85 (s, 6H)
¹³C NMR (CDCl₃, 100MHz) δ (ppm) - 159.7, 149.5, 148.1, 147.1, 146.5, 144.7, 144.4, 141.0, 140.8, 138.3, 138.0, 137.0, 136.5, 136.2, 135.1, 134.5, 132.8, 129.8, 129.4, 128.8, 128.6, 127.8, 127.4, 127.1, 126.8, 126.3, 126.1, 126.0, 125.1, 124.0, 122.9, 122.2, 121.7, 120.4, 119.9, 119.5, 118.7, 118.6, 117.8, 116.9, 115.4, 114.1, 113.6, 112.7, 109.0, 69.5, 44.9, 24.5

### Example 1

An anode was prepared by cutting a 15 Ω·cm² (1200A) ITO Corning™ glass substrate into a size of 50 mm×50 mm×0.7 mm, ultrasonic cleaning the substrate using isopropyl alcohol and pure water for 5 minutes each, and then irradiating the substrate under UV light for 30 minutes and exposing it to ozone to clean. The prepared anode was then installed in a vacuum deposition apparatus.

2-TNATA, was vacuum-deposited on the anode to a thickness of 600 Å to form a HIL, and Compound 1 as a hole transporting compound was vacuum-deposited on the HIL to a thickness of 300 Å to form a HTL.

A green fluorescent host Alq₃ and a green fluorescent dopant C545T were deposited simultaneously with a weight ratio of 98:2 on the HTL to form an EML with a thickness of 300 Å.

Next, Alq₃ was deposited on the EML to a thickness of 300 Å to form an ETL, and LiF was deposited to a thickness of 10Å on the ETL to form an EIL. Finally, Al was vacuum-deposited on the EIL to a thickness of 3000 Å to form a LiF/Al electrode (cathode), thereby completing the manufacture of an organic light emitting device.

The device had a driving voltage of 6.42 V, a high emission brightness of 8,303 cd/m², color coordinates of (0.310, 0.644), and an emission efficiency of 16.6 cd/A, at a current density of 50 mA/cm².

### Example 2

An organic light emitting device was manufactured in the same manner as in Example 1, except that Compound 6 was used instead of Compound 1 in forming the HTL.

The device had a driving voltage of 6.28 V, a high emission brightness of 8,485 cd/cm², color coordinates of (0.310, 0.643), and an emission efficiency of 17.0 cd/A, at a current density of 50 mA/cm².

### Example 3

An organic light emitting device was manufactured in the same manner as in Example 1, except that Compound 7 was used instead of Compound 1 in forming the HTL.

The device had a driving voltage of 6.16 V, a high .emission brightness of 8,590 cd/cm², color coordinates of (0.309, 0.642), and an emission efficiency of 17.2 cd/A, at a current density of 50 mA/cm².

### Example 4

An organic light emitting device was manufactured in the same manner as in Example 1, except that Compound 9 was used instead of Compound 1 in forming the HTL.

The device had a driving voltage of 6.45 V, a high emission brightness of 7,634 cd/m², color coordinates of (0.310, 0.642), and an emission efficiency of 15.3 cd/A, at a current density of 50 mA/cm².

### Example 5

An organic light emitting device was manufactured in the same manner as in Example 1, except that Compound 22 was used instead of Compound 1 in forming the HTL.

The device had a driving voltage of 6.34 V, a high emission brightness of 8,036 cd/m², color coordinates of (0.309, 0.641), and an emission efficiency of 16.1 cd/A, at a current density of 50 mA/cm².

### Example 6

An organic light emitting device was manufactured in the same manner as in Example 1, except that Compound 46 was used instead of Compound 1 in forming the HTL.

The device had a driving voltage of 6.39 V, a high emission brightness of 8,126 cd/m², color coordinates of (0.310, 0.643), and an emission efficiency of 16.3 cd/A, at a current density of 50 mA/cm².

### Example 7

An organic light emitting device was manufactured in the same manner as in Example 1, except that Compound 60 was used instead of Compound 1 in forming the HTL.

The device had a driving voltage of 6.29 V, a high emission brightness of 7,914 cd/m², color coordinates of (0.311, 0.644), and an emission efficiency of 15.8 cd/A, at a current density of 50 mA/cm².

### Example 8

An organic light emitting device was manufactured in the same manner as in Example 1, except that Compound 72 was used instead of Compound 1 in forming the HTL.

The device had a driving voltage of 6.24 V, a high emission brightness of 8,393 cd/m², color coordinates of (0.309, 0.643), and an emission efficiency of 16.8 cd/A, at a current density of 50 mA/cm².

### Example 9

An organic light emitting device was manufactured in the same manner as in Example 1, except that Compound 74 was used instead of Compound 1 in forming the HTL.

The device had a driving voltage of 6.30 V, a high emission brightness of 8,090 cd/m², color coordinates of (0.309, 0.641), and an emission efficiency of 16.2 cd/A, at a current density of 50 mA/cm².

### Example 10

An organic light emitting device was manufactured in the same manner as in Example 1, except that Compound 83 was used instead of Compound 1 in forming the HTL.

The device had a driving voltage of 6.34 V, a high emission brightness of 7,898 cd/m², color coordinates of (0.310, 0.642), and an emission efficiency of 15.8 cd/A, at a current density of 50 mA/cm².

### Comparative Example 1

An organic light emitting device was manufactured in the same manner as in Example 1, except that 4,4'-bis[N-(1-naphthyl)-N-phenylamino]biphenyl (NPB) was used instead of Compound 1 in forming the HTL.

The device had a driving voltage of 7.45 V, a high emission brightness of 6,102 cd/m², color coordinates of (0.309, 0.642), and an emission efficiency of 12.2 cd/A, at a current density of 50 mA/cm².

Each of the organic light emitting devices of Examples 1 through 10 had a driving voltage of about at least 1 V lower than the organic light emitting device of Comparative Example 1, and improved efficiency, as well as excellent I-V-L characteristics.

Half life-span values of the organic light emitting devices of Examples 1, 3, 4, 6, 7, and 9 and Comparative Example 1 are shown in Table 1 below:

**Table 1**

| | Hole transporting material | Half life-span (hr @ 100mA/cm²) |
|---|---|---|
| Example 1 | Compound 1 | 268 hr |
| Example 3 | Compound 7 | 291 hr |
| Example 4 | Compound 9 | 258 hr |
| Example 6 | Compound 46 | 295 hr |
| Example 7 | Compound 60 | 353 hr |
| Example 9 | Compound 74 | 321 hr |
| Comparative Example 1 | NPB | 113 hr |

Referring to Table 1, it can be seen that the organic light emitting devices of Examples 1, 3, 4, 6, 7, and 9 have improved life-span compared with the organic light emitting device of Comparative Example 1.

As described above, the amine-based compound represented by Formula 1 has excellent electrical properties and charge transporting capability, and thus is suitable for use as a hole injecting material, hole transporting material, and/or a light emitting material of an organic light emitting device.

While the present invention has been described with reference to certain exemplary embodiments, it will be understood by those of ordinary skill in the art that various changes may be made to the described embodiments without departing from the scope of the present invention, as defined in the appended claims and their equivalents.

## Claims

1. An amine-based compound represented by Formula 1, 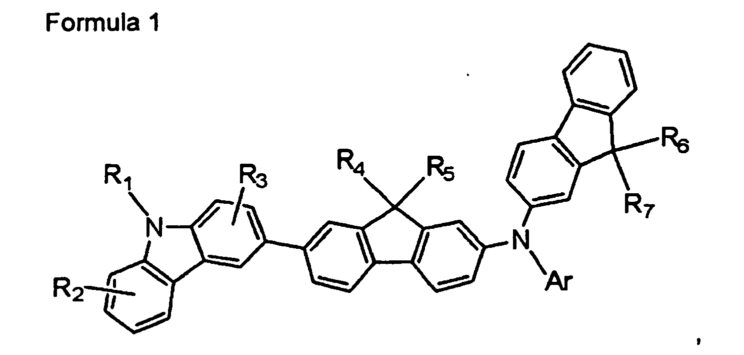 wherein Ar is a substituted or unsubstituted C₆-C₃₀ aryl group, a substituted or unsubstituted C₆-C₂₀ aryloxy group, a substituted or unsubstituted C₄-C₂₀ heteroaryl group, or a substituted or unsubstituted C₄-C₂₀ condensed polycyclic group,
R₁ is hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ heteroaryl group, or a substituted or unsubstituted C₄-C₂₀ condensed polycyclic group,
each of R₂ and R₃ is independently hydrogen, a substituted or unsubstituted C₁-C₂₀ alkyl group, a substituted or unsubstituted C₁-C₂₀ alkoxy group, a substituted or unsubstituted C₆-C₃₀ aryl group, a substituted or unsubstituted C₄-C₂₀ condensed polycyclic group, fluorine, a cyano group, or an amino group, and
each of R₄, R₅, R₆, and R₇ is independently hydrogen, a substituted or unsubstituted C₁-C₂₀ alkyl group, a substituted or unsubstituted C₁-C₂₀ alkoxy group, or a substituted or unsubstituted C₆-C₃₀ aryl group.

2. An amine-based compound according to claim 1, wherein Ar is a substituted or unsubstituted C₆-C₁₆ aryl group, or a substituted or unsubstituted C₄-C₁₆ heteroaryl group.

3. An amine-based compound according to claim 1, wherein Ar is a phenyl group, a biphenyl group, a terphenyl group, a naphthyl group, a pentalenyl group, an indenyl group, an anthracenyl group, an azulenyl group, a heptalenyl group, an acenaphthylenyl group, a fluorenyl group, a penanthrenyl group, a triphenylenyl group, a naphthacenyl group, a phenalenyl group, a pyrenyl group, a pentaphenyl group, a hexaphenyl group, a pyridinyl group, a quinolinyl group, a pyrazinyl group, a pyrimidinyl group, or a carbazolyl group, each of which may be unsubstituted or substituted with a halogen atom, a cyano group, an amino group, a (C₁-C₁₀ alkyl)amino group, a di(C₁-C₁₀ alkyl)amino group, a (C₆-C₁₄ aryl)amino group, a di(C₆-C₁₄ aryl)amino group, a C₁-C₁₀ alkyl group, a C₁-C₁₀ alkoxy group, a C₆-C₁₄ aryloxy group, a C₆-C₁₄ aryl group, or a C₆-C₁₄ aryl group substituted with a C₆-C₁₄ aryl substituent.

4. An amine-based compound according to claim 1, wherein Ar is represented by any of Formulae 2a through 2q: 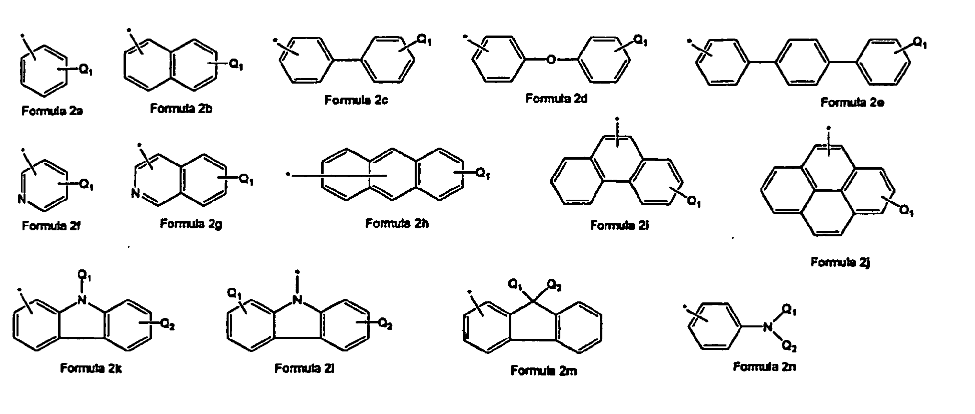 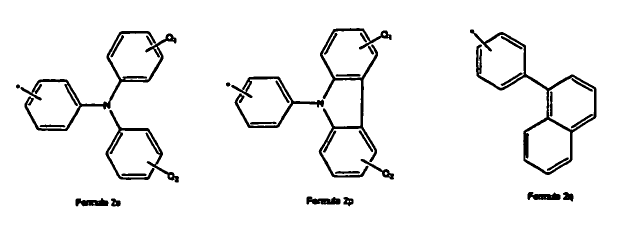 wherein each of Q₁ and Q₂ is independently hydrogen, fluorine, a cyano group, an amino group, a C₁-C₁₀ alkyl group, a C₁-C₁₀ alkoxy group, a C₆-C₁₄ aryl group, or a C₆-C₁₄ aryl group substituted with a C₆-C₁₄ aryl group, and * represents a binding site with the N of Formula 1.

5. An amine-based compound according to any of claims 1 to 4, wherein R₁ is a substituted or unsubstituted C₆-C₁₄ aryl group.

6. An amine-based compound according to any of claims 1 to 4, wherein R₁ is a phenyl group, a naphthyl group, a pentalenyl group, an indenyl group, an anthracenyl group, an azulenyl group, a heptalenyl group, an acenaphthylenyl group, a fluorenyl group, a penanthrenyl group, a triphenylenyl group, a naphthacenyl group, a penalenyl group, a pyrenyl group, a pentaphenyl group, or a hexaphenyl group, each of which may be unsubstituted or substituted with a halogen atom, a cyano group, an amino group, a (C₁-C₁₀ alkyl)amino group, a di(C₁-C₁₀ alkyl)amino group, a (C₆-C₁₄ aryl)amino group, a di(C₆-C₁₄ aryl)amino group, a C₁-C₁₀ alkyl group, a C₁-C₁₀ alkoxy group, a C₆-C₁₄ aryloxy group, a C₆-C₁₄ aryl group, or a C₆-C₁₄ aryl group substituted with a C₆-C₁₄ aryl substituent.

7. An amine-based compound according to any of claims 1 to 4, wherein R₁ is a phenyl group, a naphthyl group, a halophenyl group, or a phenyl group substituted with a phenyl substituent.

8. An amine-based compound according to any of claims 1 to 7, wherein each of R₂ and R₃ is independently hydrogen, fluorine, a cyano group, an amino group, a C₁-C₁₀ alkyl group, or a C₁-C₁₀ alkoxy group.

9. An amine-based compound according to any of claims 1 to 8, wherein each of R₄ through R₇ is independently hydrogen, a C₁-C₁₀ alkyl group, a C₁-C₁₀ alkoxy group, or a C₆-C₁₄ aryl group.

10. An amine-based compound according to claim 1, wherein Formula 1 is represented by any one of the following formulae:

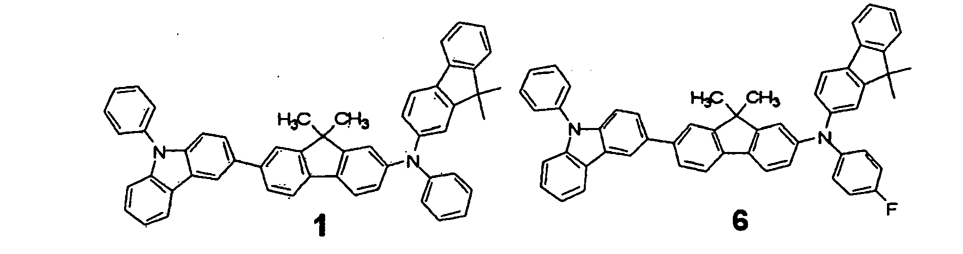

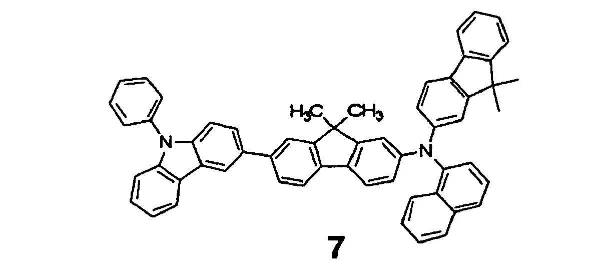

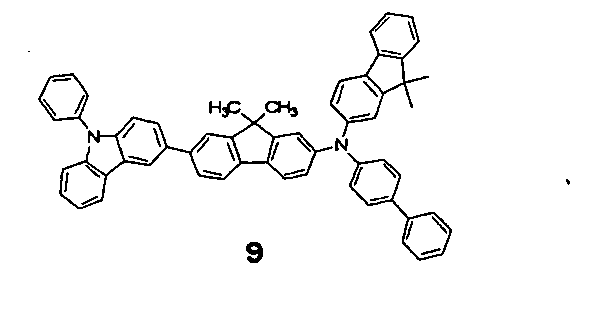

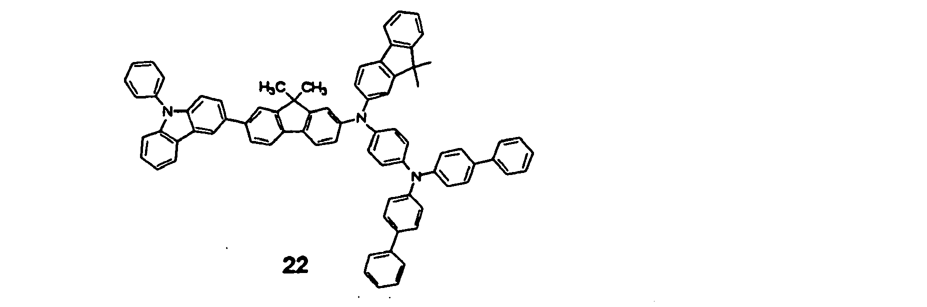

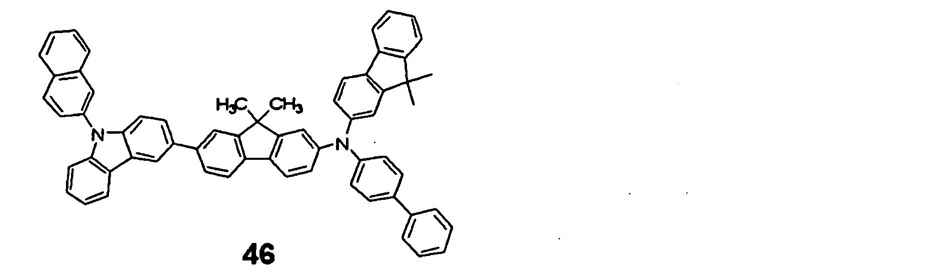

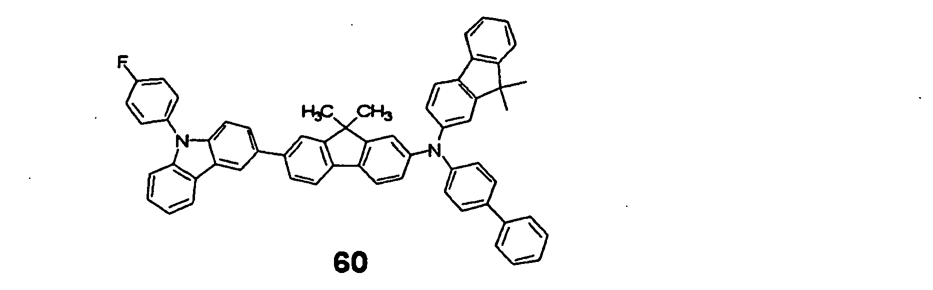

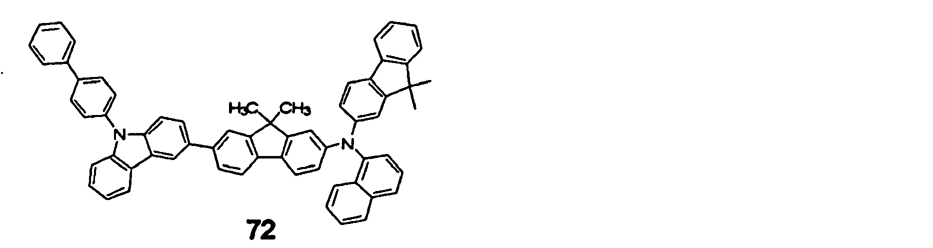

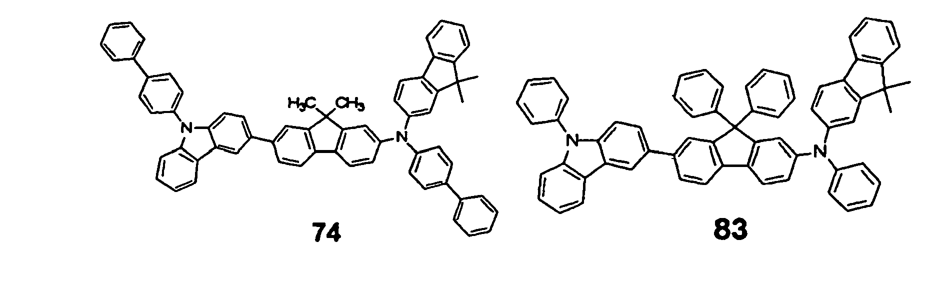

11. An organic light emitting device comprising: a first electrode; a second electrode; and an organic film between the first electrode and the second electrode, wherein the organic film comprises the amine-based compound according to any of claims 1 to 10.

12. An organic light emitting device according to claim 11, wherein the organic film comprises a hole injection layer or a hole transport layer.

13. An organic light emitting device according to claim 11, wherein the organic film comprises a single film having both hole injection and hole transport capabilities.

14. An organic light emitting device according to claim 11, wherein the organic film comprises an emissive layer.

15. An organic light emitting device according to claim 11, wherein the organic film further comprises a layer selected from the group consisting of hole injection layers, hole transport layers, electron blocking layers, emissive layers, hole blocking layers, electron transport layers, electron injection layers, and combinations thereof.

16. An organic light emitting device according to claim 15, wherein the organic light emitting device has a first electrode/hole injection layer/emissive layer/second electrode structure, a first electrode/hole injection layer/hole transport layer/emissive layer/electron transport layer/second electrode structure, or a first electrode/hole injection layer/hole transport layer/emissive layer/electron transport layer/electron injection layer/second electrode structure.

17. A flat panel display device comprising the organic light emitting device according to any of claims 11 to 16 and a thin-film transistor, wherein the first electrode of the organic light emitting device is electrically connected to a source electrode or a drain electrode of the thin-film transistor.

## Patentansprüche

1. Aminbasierte Verbindung, die durch Formel 1 dargestellt wird 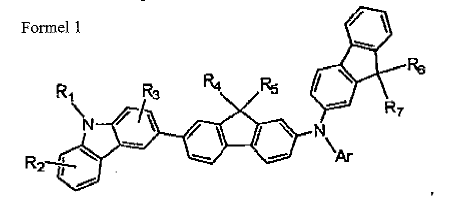 wobei Ar eine substituierte oder nichtsubstituierte C₆-C₃₀-Arylgruppe, eine substituierte oder nichtsubstituierte C₆-C₂₀-Aryloxygruppe, eine substituierte oder nichtsubstituierte C₄-C₂₀-Heteroarylgruppe oder eine substituierte oder nichtsubstituierte kondensierte polycyclische C₄-C₂₀-Gruppe ist
R₁ Wasserstoff, eine substituierte oder nichtsubstituierte C₁-C₂₀-Alkylgruppe, eine substituierte oder nichtsubstituierte C₁-C₂₀-Alkoxygruppe, eine substituierte oder nichtsubstituierte C₆-C₃₀-Arylgruppe eine substituierte oder nichtsubstituierte C₄-C₂₀-Heteroarylgruppe oder eine substituierte oder nichtsubstituierte kondensierte polycyclische C₄-C₂₀-Gruppe ist,
R₂ und R₃ jeweils unabhängig voneinander Wasserstoff, eine substituierte oder nichtsubstituierte C₁-C₂₀-Alkylgruppe, eine substituierte oder nichtsubstituierte C₁-C₂₀-Alkoxygruppe, eine substituierte oder nichtsubstituierte C₆-C₃₀-Arylgruppe, eine substituierte oder nichtsubstituierte kondensierte polycyclische C₄-C₂₀-Gruppe, Fluor, eine Cyanogruppe oder eine Aminogruppe sind, und
R₄, R₅, R₆, und R₇ jeweils unabgängig voneinander Wasserstoff, eine substituierte oder nichtsubstituierte C₁-C₂₀-Alkylgruppe, eine substituierte oder nichtsubstituierte C₁-C₂₀-Alkoxygruppe oder eine substituierte oder nichtsubstituierte C₆-C₃₀-Arylgruppe sind.

2. Aminbasierte Verbindung nach Anspruch 1, wobei Ar eine substituierte oder nichtsubstituierte C₆-C₁₆-Arylgruppe oder eine substituierte oder nichtsubstituierte C₄-C₁₆-Heteroarylgruppe ist.

3. Aminbasierte Verbindung nach Anspruch 1, wobei Ar eine Phenylgruppe, eine Biphenylgruppe, einer Terphenylgruppe, eine Naphthylgruppe, eine Pentalenylgruppe, eine Indenylgruppe, eine Anthracenylgruppe, eine Azulenylgruppe, eine Heptalenylgruppe, eine Acenaphthylenylgruppe, eine Fluorenylgruppe, eine Phenanthrenylgruppe, eine Triphenylenylgruppe, eine Naphthacenylgruppe, eine Phenylenylgruppe, eine Pyrenylgruppe, eine Pentaphenylgruppe, eine Hexaphenylgruppe, eine Pyridinylgruppe, eine Chinolinylgruppe, eine Pyrazinylgruppe, eine Pyrimidinylgruppe oder eine Carbazolylgruppe ist, die jeweils nichtsubstituiert oder mit einem Halogenatom, einer Cyanogruppe, einer Aminogruppe, einer (C₁-C₁₀-Alkyl)aminogruppe, einer Di(C₁-C₁₀-alkyl)aminogruppe, einer (C₆-C₁₄-Aryl)aminogruppe, einer Di(C₆-C₁₄-aryl)aminogruppe, einer C₁-C₁₀-Alkylgruppe, einer C₁-C₁₀-Alkoxygruppe, einer C₆-C₁₄-Aryloxygruppe, einer C₆-C₁₄-Arylgruppe oder einer mit einem C₆-C₁₄-Arylsubstituenten substituierten C₆-C₁₄-Arylgruppe substituiert sein kann.

4. Aminbasierte Verbindung nach Anspruch 1, wobei Ar durch eine der Formeln 2a bis 2q dargestellt wird: 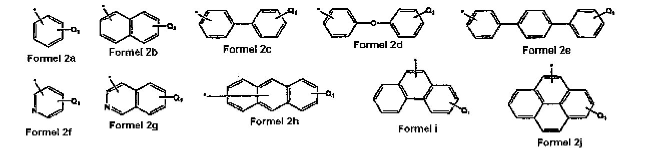 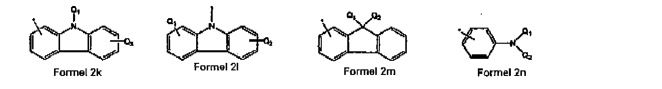 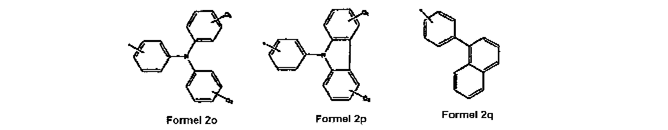 wobei Q₁ und Q₂ jeweils unabhängig voneinander Wasserstoff, Fluor, eine Cyanogruppe, eine Aminogruppe, eine C₁-C₁₀-Alkylgruppe, eine C₁-C₁₀-Alkoxygruppe, eine C₆-C₁₄-Arylgruppe oder eine mit einer C₆-C₁₄-Arylgruppe substituierte C₆-C₂₄-Arylgruppe sind und * eine Bindungsstelle mit dem N von Formel 1 darstellt.

5. Aminbasierte Verbindung nach einem der Ansprüche 1 bis 4, wobei R₁ eine substituierte oder nichtsubstituierte C₆-C₁₄-Arylgruppe ist.

6. Aminbasierte Verbindung nach einem der Ansprüche 1 bis 4, wobei R₁ eine Phenylgruppe, eine Naphthylgruppe, eine Pentalenylgruppe, eine Indenylgruppe, eine Anthracenylgruppe, eine Azulenylgruppe, eine Heptalenylgruppe, eine Acenaphthylenylgruppe, eine Fluorenylgruppe, eine Phenanthrenylgruppe, eine Triphenylenylgruppe, eine Naphthacenylgruppe, eine Phenylenylgruppe, eine Pyrenylgruppe, eine Pentaphenylgruppe oder eine Hexaphenylgruppe ist, die jeweils nichtsubstituiert oder mit einem Halogenatom, einer Cyanogruppe, einer Aminogruppe, einer (C₁-C₁₀-Alkyl)aminogruppe, einer Di(C₁-C₁₀-alkyl)aminogruppe, einer (C₆-C₁₄-Aryl)aminogruppe, einer Di(C₆-C₁₄-aryl)aminogruppe, einer C₁-C₁₀-Alkylgruppe, einer C₁-C₁₀-Alkoxygruppe, einer C₆-C₁₄-Aryloxygruppe, einer C₆-C₁₄-Arylgruppe oder einer mit einem C₆-C₁₄-Arylsubstituenten substituierten C₆-C₁₄-Arylgruppe substituiert sein kann.

7. Aminbasierte Verbindung nach einem der Ansprüche 1 bis 4, wobei R₁ eine Phenylgruppe, eine Naphthylgruppe, eine Halogenphenylgruppe oder eine mit einem Phenylsubstituenten substituierte Phenylgruppe ist.

8. Aminbasierte Verbindung nach einem der Ansprüche 1 bis 7, wobei R₂ und R₃ jeweils unabhängig voneinander Wasserstoff, Fluor, eine Cyanogruppe, eine Aminogruppe, eine C₁-C₁₀-Alkylgruppe oder eine C₁-C₁₀-Alkoxygruppe sind.

9. Aminbasierte Verbindung nach einem der Ansprüche 1 bis 8, wobei R₄ bis R₇ jeweils unabhängig voneinander Wasserstoff, eine C₁-C₁₀-Alkylgruppe, eine C₁-C₁₀-Alkoxygruppe oder eine C₆-C₁₄-Arylgruppe sind.

10. Aminbasierte Verbindung nach Anspruch 1, wobei Formen 1 durch eine der folgenden Formeln dargestellt wird:

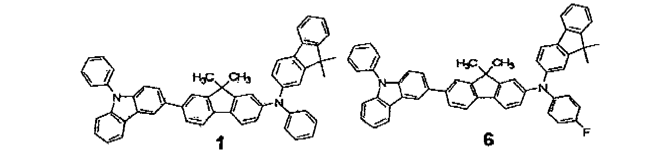

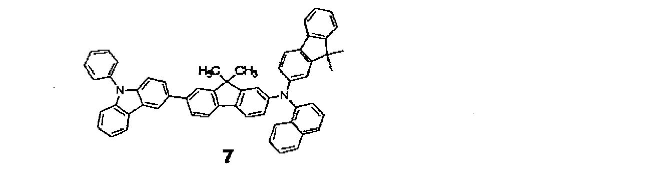

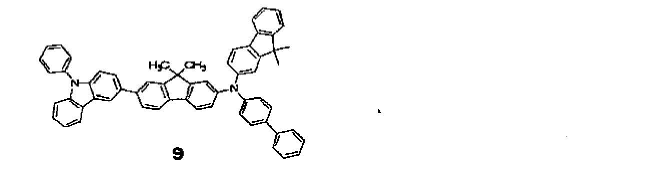

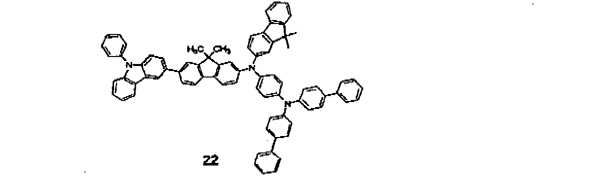

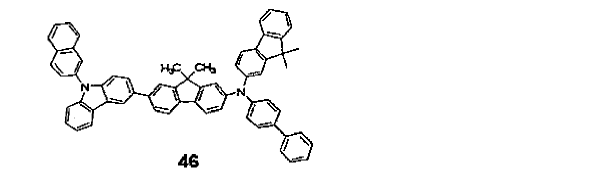

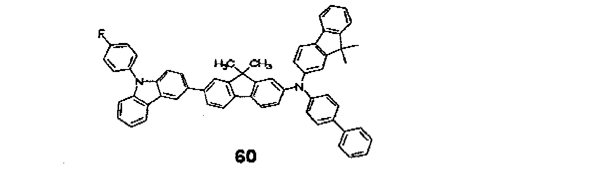

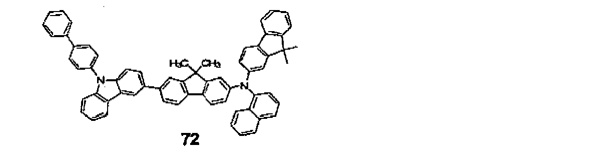

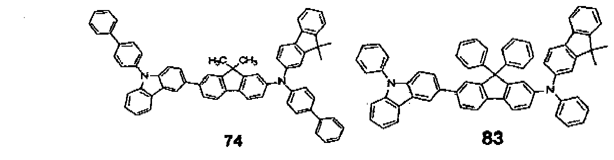

11. Organisches lichtemittierendes Element, das aufweist: eine erste Elektrode, eine zweite Elektrode und eine organische Schicht zwischen der ersten Elektrode und der zweiten Elektrode, wobei die organische Schicht die aminbasierte Verbindung nach einem der Ansprüche 1 bis 10 aufweist.

12. Organisches lichtemittierendes Element nach Anspruch 11, wobei die organische Schicht eine Löcherinjektionsschicht oder eine Löchertransportschicht aufweist.

13. Organisches lichtemittierendes Element nach Anspruch 11, wobei die organische Schicht eine einzige Schicht aufweist, die sowohl Löcherinjektions- als auch Löchertransportfähigkeiten aufweist.

14. Organisches lichtemittierendes Element nach Anspruch 11, wobei die organische Schicht eine Emissionsschicht aufweist.

15. Organisches lichtemittierendes Element nach Anspruch 11, wobei die organische Schicht ferner eine Schicht aufweist, die aus der Gruppe ausgewählt ist, die aus Löcherinjektionsschichten, Löchertransportschichten, Elektronensperrschichten, Emissionsschichten, Löchersperrschichten, Etektronentransportschichten, Elektroneninjektionsschichten und Kombinationen davon besteht.

16. Organisches lichtemittierendes Element nach Anspruch 15, wobei das organische lichtemittierende Element eine Struktur aus erster Elektrode/Löcherinjektionsschicht/Emissionsschicht/zweiter Elektrode, eine Struktur aus erster Elektrode/Löcherinjektionsschicht/Löchertransportschicht/Emissionsschicht/Elektionenttansportschicht/zweiter Elektrode oder eine Struktur aus erster Elektrode/Löcherinjektionsschicht/Löchertransportschicht/Emissionssehicht/Elektronentransportschicht/- Elektroneninjektionsschicht/ zweiter Elektrode aufweist.

17. Flachbildschirm, der das organische lichtemittierende Element nach einem der Ansprüche 11 bis 16 und einen Dünnschichttransistor aufweist, wobei die erste Elektrode des organischen lichtemittierenden Elements mit einer Source-Elektrode oder einer Drain-Elektrode des Dünnschichttransistors elektrisch verbunden ist.

## Revendications

1. Composé à base d'amine représenté par la Formule 1: 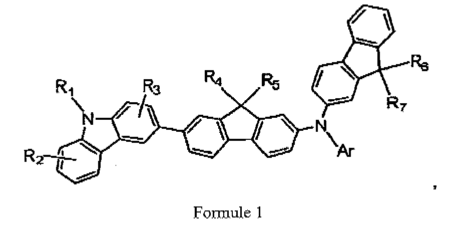 dans lequel Ar est un groupe aryle C₆-C₃₀ substitué ou non substitué, un groupe aryloxy C₆-C₂₀ substitué ou non substitué, un groupe hétéroaryle C₄-C₂₀ substitué ou non substitué ou un groupe polycyclique condensé C₄-C₂₀ substitué ou non substitué,
R₁ est un hydrogène, un groupe alkyle C₁-C₂₀ substitué ou non substitué, un groupe alcoxy C₁-C₂₀ substitué ou non substitué, un groupe aryle C₆-C₃₀ substitué ou non substitué, un groupe hétéroaryle C₄-C₂₀ substitué ou non substitué ou un groupe polycyclique condensé C₁-C₂₀ substitué ou non substitué,
chacun de R₂ et R₃ est indépendamment un hydrogène, un groupe alkyle C₁-C₂₀ substitué ou non substitué, un groupe alcoxy C₁-C₂₀ substitué ou non substitué, un groupe aryle C₆-C₃₀ substitué ou non substitué, un groupe polycyclique condensé C₄-C₂₀ substitué ou non substitué, un fluor, un groupe cyano ou un groupe amino, et
chacun de R₄, R₅, R₆ et R₇ est indépendamment un hydrogène, un groupe alkyle C₁-C₂₀ substitué ou non substitué, un groupe alcoxy C₁-C₂₀ substitué ou non substitué ou un groupe aryle C₆-C₃₀ substitué ou non substitué.

2. Composé à base d'amine selon la revendication 1, dans lequel Ar est un groupe aryle C₆-C₁₆ substitué ou non substitué ou un groupe hétéroaryle C₄-C₁₆ substitué ou non substitué.

3. Composé à base d'amine selon la revendication 1, dans lequel Ar est un groupe phényle, un groupe biphényle, un groupe terphényle, un groupe naphtyle, un groupe pentalényle, un groupe indényle, un groupe anthracényle, un groupe azulényle, un groupe heptalényle, un groupe acénaphtylényle, un groupe fluorényle, un groupe phénanthrényle, un groupe triphénylényle, un groupe naphtacényle, un groupe phénalényle, un groupe pyrényle, un groupe pentaphényle, un groupe hexaphényle, un groupe pyridinyle, un groupe quinoléinyle, un groupe pyrazinyle, un groupe pyrimidinyle ou un groupe carbazoyle, chacun pouvant être non substitué ou substitué avec un atome d'halogène, un groupe cyano, un groupe amino, un groupe (alkyle C₁-C₁₀)amino, un groupe di(alkyle C₁-C₁₀)amino, un groupe (aryle C₆-C₁₄)amino, un groupe di(aryle C₆-C₁₄)amino, un groupe alkyle C₁-C₁₀, un groupe alcoxy C₁-C₁₀, un groupe aryloxy C₆-C₁₄, un groupe aryle C₆-C₁₄ ou un groupe aryle C₆-C₁₄ substitué avec un substituant aryle C₆-C₁₄.

4. Composé à base d'amine selon la revendication 1, dans lequel Ar est représenté par l'une quelconque des formules 2a à 2q: 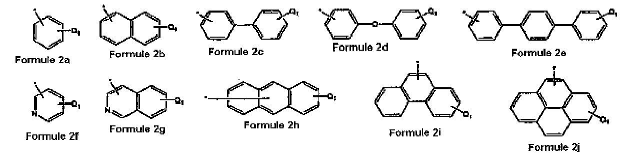 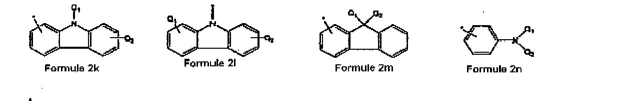 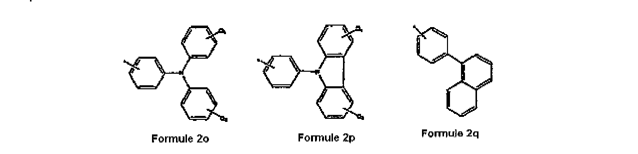 dans lesquelles chacun de Q₁ et Q₂ est indépendamment un hydrogène, un fluor, un groupe cyano, un groupe amino, un goupe alkyle C₁-C₁₀, un groupe alcoxy C₁-C₁₀, un groupe aryle C₆-C₁₄ ou un groupe aryle C₆-C₁₄ substitué avec un aryle C₆-C₁₄ et * représente un site de liaison avec N de la Formule 1.

5. Composé à base d'amine selon l'une quelconque des revendications 1 à 4, dans lequel R₁ est un groupe aryle C₆-C₁₄ substitué ou non substitué.

6. Composé à base d'amine selon l'une quelconque des revendications 1 à 4, dans lequel R₁ est un groupe phényle, un groupe naphtyle, un groupe pentalényle, un groupe indényle, un groupe anthracényle, un groupe azulényle, un groupe heptalényle, un groupe acénaphtylényle, un groupe fluorényle, un groupe phénanthrényle, un groupe triphénylényle, un groupe naphtacényle, un groupe phénatényle, un groupe pyrényle, un groupe pentaphényle ou un groupe hexaphényle, chacun pouvant être non substitué ou substitué avec un atome d'halogène, un groupe cyano, un groupe amino, un groupe (alkyle C₁-C₁₀)amino, un groupe di(alkyle C₁-C₁₀)amino, un groupe (aryle C₆-C₁₄)amino, un groupe di(aryle C₆-C₁₄)amino, un groupe alkyle C₁-C₁₀, un groupe alcoxy C₁-C₁₀, un groupe aryloxy C₆-C₁₄, un groupe aryle C₆-C₁₄ ou un groupe aryle C₆-C₁₄ substitué avec un substituant aryle C₆-C₁₄.

7. Composé à base d'amine selon l'une quelconque des revendications 1 à 4, dans lequel R₁ est un groupe phényle, un groupe naphtyle, un groupe halophényle ou un groupe phényle substitué avec un substituant phényle,

8. Composé à base d'amine selon l'une quelconque des revendications 1 à 7, dans lequel chacun de R₂ et R₃ est indépendamment un hydrogène, un fluor, un groupe cyano, un groupe amino, un groupe alkyle C₁-C₁₀ ou un groupe alcoxy C₁-C₁₀.

9. Composé à base d'amine selon l'une quelconque des revendications 1 à 8, dans lequel chacun de R₄ à R₇ est indépendamment un hydrogène, un groupe alkyle C₁-C₁₀, un groupe alcoxy C₁-C₁₀ ou un groupe aryle C₆-C₁₄.

10. Composé à base d'amine selon la revendication 1, dans lequel la Formule 1 est représentée par l'une quelconque des formules suivantes:

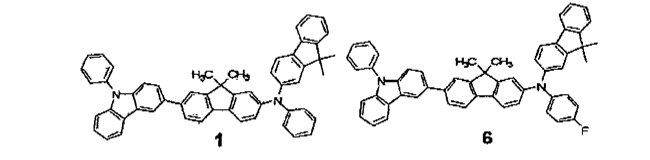

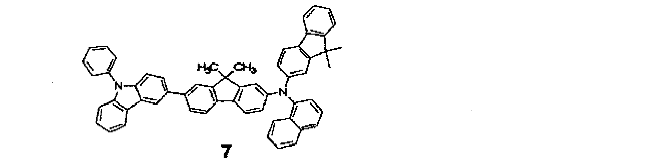

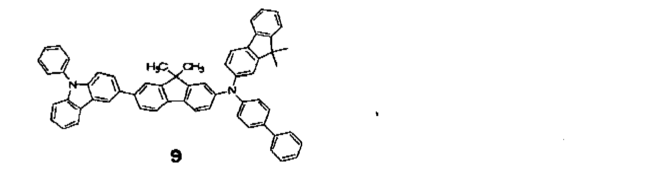

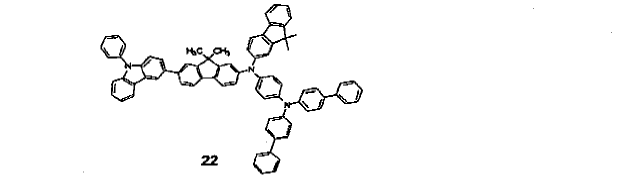

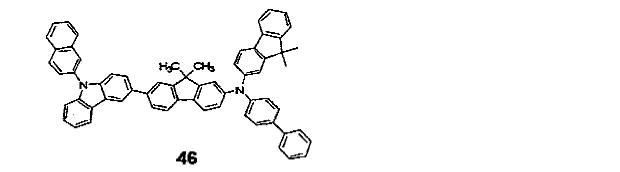

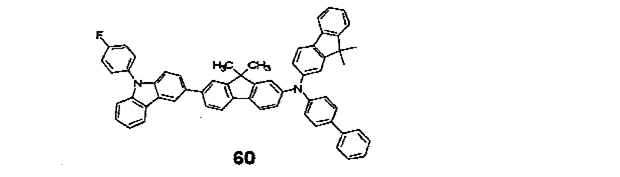

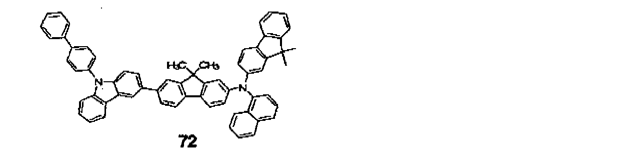

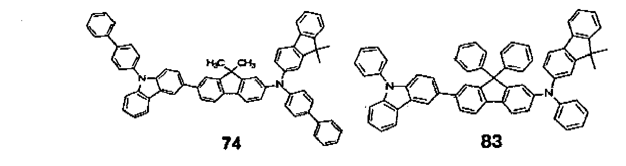

11. Dispositif électroluminescent organique comprenant: une première électrode; une deuxième électrode; et un film organique entre la première électrode et la deuxième électrode, dans lequel le film organique comprend le composé à base d'amine selon l'une quelconque des revendications 1 à 10.

12. Dispositif électroluminescent organique selon la revendication 11, dans lequel le film organique comprend une couche d'injection de trous ou une couche de transport de trous.

13. Dispositif électroluminescent organique selon la revendication 11, dans lequel le film organique comprend un seul film possédant à la fois des capacités d'injection de trous et de transport de trous.

14. Dispositif électroluminescent organique selon la revendication 11, dans lequel le film organique comprend une couche émissive.

15. Dispositif électroluminescent organique selon la revendication 11, dans lequel le film organique comprend en outre une couche choisie dans le groupe constitué de couches d'injection de trous, de couches de transport de trous, de couches de blocage d'électrons, de couches émissives, de couches de blocage de trous, de couches de transport d'électrons, de couches d'injection d'électrons et de combinaisons de celles-ci.

16. Dispositif électroluminescent organique selon la revendication 15, où le dispositif électroluminescent organique possède une structure de première électrode/couche d'injection de trous/couche émissive/deuxième électrode, une structure de première électrode/couche d'injection de trous/couche de transport de trous/couche émissive/couche de transport d'électrons/deuxième électrode ou une structure de première électrode/couche d'injection de trous/couche de transport de trous/couche émissive/couche de transport d'électrons/couche d'injection d'électrons/deuxième électrode.

17. Dispositif d'affichage à écran plat comprenant le dispositif électroluminescent organique selon l'une quelconque des revendications 11 à 16 et un transistor en couches minces, dans lequel la première électrode du dispositif électroluminescent organique est connectée électriquement à une électrode de source ou une électrode de drain du transistor en couches minces.
